(19) Europäisches Patentamt
European Patent Office
Office européen des brevets

(11) EP 4 302 863 A1

(12) **EUROPEAN PATENT APPLICATION**
published in accordance with Art. 153(4) EPC

(43) Date of publication:
**10.01.2024 Bulletin 2024/02**

(21) Application number: **22763438.3**

(22) Date of filing: **04.03.2022**

(51) International Patent Classification (IPC):
***B01D 61/02*** $^{(2006.01)}$ ***B01D 61/58*** $^{(2006.01)}$
***B01D 71/06*** $^{(2006.01)}$ ***C02F 1/04*** $^{(2023.01)}$
***C02F 1/42*** $^{(2023.01)}$ ***C02F 1/44*** $^{(2023.01)}$
***G01N 33/53*** $^{(2006.01)}$ ***G01N 33/92*** $^{(2006.01)}$

(52) Cooperative Patent Classification (CPC):
**B01D 61/02; B01D 61/58; B01D 71/06; C02F 1/04; C02F 1/42; C02F 1/44; G01N 33/53; G01N 33/579; G01N 33/92**

(86) International application number:
**PCT/JP2022/009508**

(87) International publication number:
**WO 2022/186388 (09.09.2022 Gazette 2022/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **05.03.2021 JP 2021035810**

(71) Applicants:
- **Nomura Micro Science Co., Ltd.**
  **Atsugi-shi, Kanagawa 243-0021 (JP)**
- **Sophia School Corporation**
  **Tokyo 102-8554 (JP)**

(72) Inventors:
- **KIMOTO, Hiroshi**
  **Atsugi-Shi, Kanagawa 243-0021 (JP)**
- **IIYAMA, Masamitsu**
  **Atsugi-Shi, Kanagawa 243-0021 (JP)**
- **EBISAWA, Yu**
  **Tokyo 102-8554 (JP)**
- **HASHIMOTO, Takeshi**
  **Tokyo 102-8554 (JP)**
- **HAYASHITA, Takashi**
  **Tokyo 102-8554 (JP)**

(74) Representative: **DREISS Patentanwälte PartG mbB**
**Friedrichstraße 6**
**70174 Stuttgart (DE)**

# (54) TEST SUBSTANCE DETECTION METHOD, DETECTION AGENT COMPOSITION, DETECTION APPARATUS, PURIFIED WATER PRODUCTION FACILITY, INJECTION WATER PRODUCTION FACILITY, PURIFIED WATER PRODUCTION METHOD AND INJECTION WATER PRODUCTION METHOD

(57) A test substance detection method includes: a step A in which a sample containing a test substance and water, a compound having a structure in which a response site and a recognition site are connected via a spacer, and a surfactant are added; and a step B in which the recognition site and the test substance interact and a response at the response site arising from the interaction is detected. Further, also provided are: a purified water production method and an injection water production method both using the foregoing; a detection agent composition containing a compound represented by Formula (1) and a surfactant; and a detection apparatus, a purified water production facility, and an injection water production facility, all using the foregoing.

EP 4 302 863 A1

## Description

### Technical Field

The present disclosure relates to a test substance detection method, a detection agent composition, a detection apparatus, a purified water production facility, an injection water production facility, a purified water production method, and an injection water production method.

### Background Art

Many methods of detecting trace components contained in food, water, soil, blood, and the like are known, and detection methods suitable for detection targets have been conventionally studied.

As the trace components, for example, a pyrogenic substance (endotoxin) and the like are known, and in the production of injection water (WFI, Water for Injection), sterilized purified water obtained by sterilizing purified water is subjected to a predetermined endotoxin test.

The purified water is obtained by purifying normal water by distillation, ion exchange using an ion exchange resin tower, an electrodeionization device (EDI), or the like, reverse osmosis or ultrafiltration, an ultraviolet irradiation device (UV), or a combination thereof, and is used not only as a raw material of a preparation but also for preparation of a reagent and the like.

As a WFI production facility, for example, Japanese Patent Application Laid-Open (JP-A) No. 2019-025456 describes an injection water production apparatus including a polymer membrane filtration device that filters treatment target water obtained by treating raw water by a combination of a reverse osmosis membrane treatment and an ion exchange treatment and a fine particle measurement device that measures the number of fine particles in permeated water of the polymer membrane filtration device.

Endotoxins are lipopolysaccharides that are cell wall components of gram-negative bacteria, and are representative pyrogens unevenly distributed in living environments. Since actions such as fever, septic shock, multiple organ failure, and tachycardia occur in a case in which an endotoxin enters blood, strict management is required in the manufacture of pharmaceutical products and medical devices, particularly liquids directly introduced into a living body and medical devices such as pharmaceutical water, syringes, artificial organs, and dialysis membranes. For example, in the control standard of injection water in "Japanese Pharmacopoeia (JP17) Quality Conformance Test", the endotoxin is defined as less than 0.25 EU/mL.

As an endotoxin detection method, for example, detection of an endotoxin by an electrochemical method described in JP-ANos. 2016-151482 and 2007-093378 is disclosed.

## SUMMARY OF INVENTION

### Technical Problem

Although many methods of detecting trace components in a sample are known, there are cases in which any of the methods has some problems such as a need for a large amount of specimen and unmeasurable situations due to the presence of interfering substances, and further development is required.

WFI production requires a high degree of removal of an endotoxin from purified water produced in a purified water production facility by an endotoxin removal facility such as a distiller or a polymer membrane filtration device (preferably an ultrafiltration membrane device).

In a case in which an abnormality occurs in endotoxin removal capability in an endotoxin removal facility, the endotoxin concentration in WFI easily increases, and the endotoxin removal facility is unsuitable for production of an injection solution. In particular, in recent years, in order to reduce energy consumption, there is an increasing tendency to employ a polymer membrane filtration device instead of a conventional distiller as such a facility. However, since a resin material is used as a polymer membrane in a polymer membrane filtration device, there is a concern that the resin material deteriorates every time heat sterilization is performed, for example. This concern is a factor that hinders adoption of a polymer membrane filtration device in an endotoxin removal facility.

Therefore, in order to stably produce WFI of required water quality, for example, it is necessary to monitor a change in endotoxin removal capability and perform maintenance such as immediate replacement or repairing of a component such as a polymer membrane before an abnormality occurs in an endotoxin removal facility. However, a method capable of rapidly detecting a change in endotoxin removal capability has not yet been realized.

Detection of the endotoxin has been performed by a limulus test utilizing coagulation of blood cell components of horseshoe crabs by the endotoxin. In the limulus test, it is currently standard to perform the test using a lysate reagent extracted from the blood of a horseshoe crab, but it takes from 1 to 2 hours for the test result to be obtained. Although

an online measurement device using a limulus test has also been devised, it is difficult to obtain a practical quantitation limit, and quantitativity and reproducibility are poor. For example, since the reagent itself is biologically derived, its performance may vary depending on the lot of reagents. For this reason, a detection method capable of obtaining test results more quickly and performing online measurement with practical accuracy has been desired.

In order to obtain a lysate reagent, it is necessary to capture a wild organism such as a horseshoe crab and collect blood, and thus, from the viewpoint of animal welfare, development of an alternative means that does not use blood of a horseshoe crab has been desired.

Detection of an endotoxin by an electrochemical method has many problems such as a production technique or mass productivity of an electrode used for detection, required maintenance frequency or measurement accuracy, a quantitation limit, and rapidity, in order to particularly obtain a practical quantitation limit, and has not been put into practical use.

An object of an embodiments of the disclosure is to provide a test substance detection method capable of detecting a test substance with high sensitivity, and a purified water production method and an injection water production method both using the test substance detection method. Another object of the disclosure is to provide a detection agent capable of detecting a test substance with high sensitivity, and a detection apparatus, a purified water production facility, and an injection water production facility, all using the detection agent.

Solution to Problem

Means for solving the above problems include the following aspects.

<1> A test substance detection method including:

a step A of mixing a sample containing a test substance and water, a compound having a structure including a response site and a recognition site, and a surfactant; and
a step B of detecting a response at the response site arising from interaction between the recognition site and the test substance.

<2> The test substance detection method according to <1>, wherein the compound has a structure in which the response site and the recognition site are connected via a spacer.
<3> The test substance detection method according to <1> or <2>, wherein the compound is a compound represented by the following Formula (1):

A-L-Y Formula (1)

wherein A represents a response site which is a group containing a conjugated multiple bond, L represents a single bond or a spacer having a chain length of from 1 to 10 atoms, and Y represents a recognition site which is a group interacting with a test substance.
<5> The test substance detection method according to <3> or <4>, wherein Y in Formula (1) is a metal coordinating group or a group containing an acid group.
<6> The test substance detection method according to <5>, wherein the metal coordinating group or the group containing an acid group is a group represented by the following Formula (Y1) or Formula (Y2):

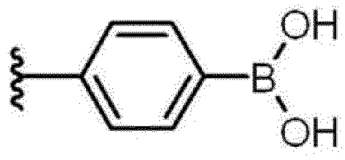

Formula (Y1)

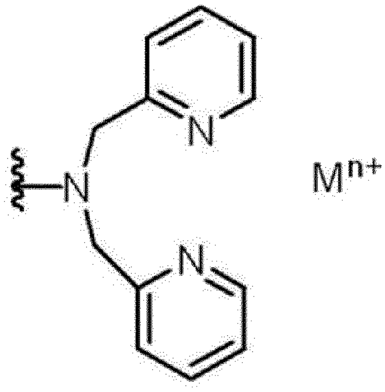

Formula (Y2)

wherein a wavy line represents a linking site with L in Formula (1), $M^{n+}$ represents a metal ion, and n represents a natural number.

<7> The test substance detection method according to <6>, wherein the metal ion is a copper ion ($Cu^{2+}$), a nickel ion ($Ni^{2+}$), or a cobalt ion ($Co^{2+}$).

<8> The test substance detection method according to any one of <1> to <7>, wherein an HLB value of the surfactant is from 6 to 40.

<9> The test substance detection method according to any one of <1> to <8>, wherein the surfactant is a cationic surfactant, an amphoteric surfactant, or a non-ionic surfactant.

<10> The test substance detection method according to any one of <1> to <9>, wherein the compound is a compound capable of forming a molecular assembly with the surfactant.

<11> The test substance detection method according to any one of <1> to <10>, wherein the test substance contains at least one of a phospholipid or a glycolipid.

<12> The test substance detection method according to any one of <1> to <11>, wherein the test substance is an endotoxin, and
the compound is a compound represented by the following Formula (1A) or C4-APB:

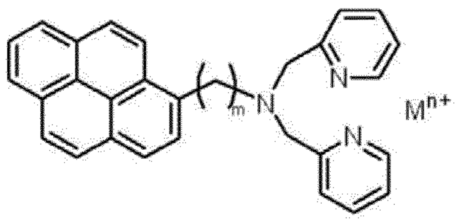

Formula (1A)

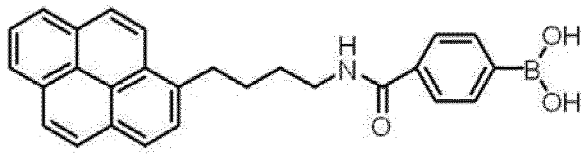

(C4-APB)

wherein m represents from 0 to 6, $M^{n+}$ represents a metal ion, and n represents a natural number.

<13> A detection agent composition containing:

a compound represented by the following Formula (1); and
a surfactant:

A-L-Y Formula (1)

wherein A represents a response site which is a group containing a conjugated multiple bond, L represents a single bond or a spacer having a chain length of from 1 to 10 atoms, and Y represents a recognition site which is an organic group.

<14> The detection agent composition according to <13>, wherein the L is a linear saturated alkyl chain.

<15> The detection agent composition according to <13> or <14>, wherein Y in Formula (1) is a metal coordinating group or a group containing an acid group.

<16> The detection agent composition according to <15>, wherein the metal coordinating group or the group containing an acid group is a group represented by the following Formula (Y1) or Formula (Y2):

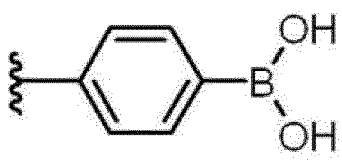

Formula (Y1)

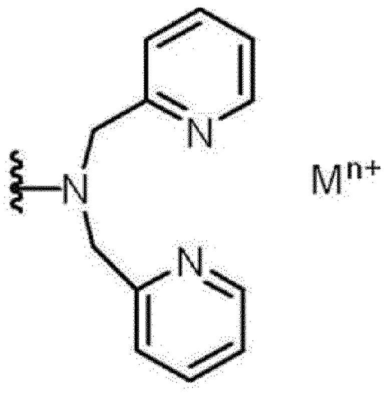

Formula (Y2)

wherein a wavy line represents a linking site with L in Formula (1), $M^{n+}$ represents a metal ion, and n represents a natural number.

<17> The detection agent composition according to <16>, wherein the metal ion is a copper ion ($Cu^{2+}$), a nickel ion ($Ni^{2+}$), or a cobalt ion ($Co^{2+}$).

<18> The detection agent composition according to any one of <13> to <17>, wherein an HLB value of the surfactant is from 6 to 40.

<19> The detection agent composition according to any one of <13> to <18>, wherein the surfactant is a cationic surfactant, an amphoteric surfactant, or a non-ionic surfactant.

<20> A detection apparatus including:

a specimen introduction unit into which a sample containing a test substance and water is introduced;
a supply unit supplying the detection agent composition according to any one of <13> to <19> to the sample;
a reaction unit in which the sample and the detection agent composition react with each other; and
a detection unit detecting the reaction.

<21> A purified water production facility including:

a purified water production unit using at least one of a reverse osmosis membrane device, which performs reverse osmosis filtration of raw water, or an ion exchange device, which performs ion exchange of raw water, to obtain purified water from the raw water;
a specimen collection unit collecting a specimen of the test subject from the purified water; and
the detection apparatus according to <20>.

<22> An injection water production facility including:

a purified water production unit using at least one of a reverse osmosis membrane device, which performs reverse osmosis filtration of raw water, or an ion exchange device, which performs ion exchange of raw water, to obtain purified water from the raw water;
an injection water production unit using a polymer membrane filtration device filtering the purified water or a distiller distilling the purified water to obtain injection water from the purified water;
an injection water tank maintaining the injection water in a heated state and storing the injection water;
a delivery line delivering the injection water stored in the injection water tank to a predetermined use place;
a specimen collection unit collecting a specimen of the test subject from the injection water; and
the detection apparatus according to <20>.

<23> The injection water production facility according to <22>, wherein the specimen collection unit collects the specimen directly from the injection water tank or from the delivery line on a downstream side of the injection water production unit and on an upstream side of the injection water tank.

<24> A purified water production method including:

a step of treating raw water by at least one of reverse osmosis filtration or ion exchange to obtain purified water; and

a measurement step of measuring a specimen of a test subject collected from the purified water using the detection method according to any one of <1> to <12>.

<25> The purified water production method according to <24>, wherein the measurement step includes a measurement step of measuring a specimen of a test subject collected from the purified water using the detection apparatus according to <20>.

<26> An injection water production method including:

a step of treating raw water by at least one of reverse osmosis filtration or ion exchange to obtain purified water;

a step of obtaining injection water from the purified water by filtration of the purified water using polymer membrane filtration or by distillation of the purified water; and

a measurement step of measuring a specimen of the test subject collected from the injection water using the detection method according to any one of <1> to <12>.

<27> The injection water production method according to <25>, wherein the measurement step includes a measurement step of measuring a specimen of a test subject collected from the purified water using the detection apparatus according to <20>. Advantageous Effects of Invention

According to an embodiment according to the disclosure, there are provided a test substance detection method capable of detecting a test substance with high sensitivity, and a purified water production method and an injection water production method both using the test substance detection method. According to another embodiment of the disclosure, there are provided a detection agent capable of detecting a test substance with high sensitivity, and a detection apparatus, a purified water production facility, and an injection water production facility, all using the detection agent.

BRIEF DESCRIPTION OF DRAWINGS

Fig. 1 is a schematic view of an example of a compound used in a test substance detection method according to the disclosure.

Fig. 2 is a schematic view illustrating a configuration of an example of a detection apparatus according to the disclosure.

Fig. 3 is a schematic view of a schematic configuration of an example of a purified water production facility and an injection water production facility according to the disclosure.

Fig. 4 is a graph showing a fluorescence spectrum change in a case in which an endotoxin is added to $Cu^{2+}$-dpa-C4Py/DDAB.

Fig. 5 is a graph of a calibration curve of a fluorescence intensity at a wavelength of 377 nm with respect to an endotoxin concentration.

Fig. 6 is a graph showing a change in fluorescence intensity in a case in which a surfactant: DDAB at each concentration is added to a sample containing $Cu^{2+}$-dpa-C4Py and an endotoxin.

Fig. 7 is a graph showing a fluorescence spectrum change in a case in which an endotoxin is added to $Zn^{2+}$-dpa-C4Py/DDAB.

Fig. 8 is a graph showing a fluorescence spectrum change in a case in which an endotoxin is added to $Cu^{2+}$-dpa-C4Py/CTAB.

Fig. 9 is a graph of a calibration curve of a fluorescence intensity at a wavelength of 377 nm with respect to an endotoxin concentration.

Fig. 10 is a graph showing a change in fluorescence intensity in a case in which a surfactant: CTAB at each concentration is added to a sample containing $Cu^{2+}$-dpa-C4Py and an endotoxin.

DESCRIPTION OF EMBODIMENTS

Hereinafter, contents according to the disclosure will be described in detail. While the description of the constituents described below will occasionally be made based on representative embodiments of the disclosure, the disclosure is not limited to such embodiments.

In the disclosure, a numerical range that has been indicated by use of "to" indicates the range that includes the numerical values which are described before and after "to", as a minimum value and a maximum value, respectively.

In a numerical range described in a stepwise manner in the disclosure, an upper limit value or a lower limit value described in one numerical range may be replaced with an upper limit value or a lower limit value described in another numerical range described in a stepwise manner. In a numerical range described in the disclosure, an upper limit value or a lower limit value described in one numerical range may be replaced with a value shown in Examples.

In the disclosure, a combination of two or more preferred aspects is a more preferred aspect.

In a case in which a plurality of substances corresponding to each component are present in a composition, the content of each component in the composition in the disclosure means the total content of the plurality of substances present in the composition unless otherwise specified.

In the disclosure, raw water may be municipal water, well water, river water, industrial water, or the like, may be water produced by water treatment apparatuses such as a purified water production apparatus and an injection water production apparatus, or may be water in a process in the middle of treatment by these water treatment apparatuses.

In the disclosure, the total solid content refers to the total mass of components excluding a solvent from the total composition of the composition. In the disclosure, the solid content is a solid content at 25°C.

In the disclosure, the term "step" includes not only an independent step but also a step by which an intended action of the step is achieved, though the step cannot be clearly distinguished from other steps.

(Test Substance Detection Method)

A test substance detection method according to the disclosure (hereinafter, also simply referred to as "detection method according to the disclosure") includes a step A of mixing a sample containing a test substance and water, a compound having a structure including a response site and a recognition site, and a surfactant; and a step B of detecting a response at the response site arising from interaction between the recognition site and the test substance.

As a result of intensive studies by the present disclosers, it has been found that, with the above configuration, the detection method according to the disclosure can detect a test substance with high sensitivity. Although the detailed mechanism by which the above effect can be obtained is unknown, it is presumed as follows.

It is presumed that, by adding a compound having a structure including a response site and a recognition site (hereinafter, also referred to as "specific compound") and a surfactant to a sample containing a test substance and water, the surfactant and the specific compound form a molecular assembly (for example, micelles, vesicles, and the like) in the sample containing water, and the test substance and the specific compound are incorporated into the molecular assembly, specifically, for example, the specific compound is incorporated into a molecular layer of the surfactant forming a surface of a spherical or cylindrical molecular assembly or embedded inside the molecular assembly, thereby detecting the test substance with high sensitivity. Since the test substance and the specific compound are incorporated into the molecular assembly, the test substance is concentrated, and further, in the molecular assembly, the recognition site of the specific compound can be in a positional relationship in which the recognition site is easy to stably interact with the test substance, so that the test substance can be detected with high sensitivity.

The detection method according to the disclosure can quickly and easily detect the test substance by including the above steps.

By selectively forming a molecular assembly of the test substance and the surfactant, the test substance can be selectively detected from a sample (specimen) in a state where the test substance and a substance other than the test substance are mixed, so that the test substance can be detected with high sensitivity. The detection method according to the disclosure can be suitably used for detecting a test substance forming a molecular assembly in water from the viewpoint that a test substance can be detected with high sensitivity.

Hereinafter, each step of the detection method according to the disclosure will be described.

<Step A>

The step A included in the detection method according to the disclosure is a step of mixing a sample containing a test substance and water, a compound having a structure including a response site and a recognition site, and a surfactant.

«Specific Compound»

The specific compound used in the step A has a structure including a response site and a recognition site.

The recognition site is not particularly limited as long as it has a structure that interacts with the test substance, but from the viewpoint of detecting a test substance with high sensitivity, the recognition site is preferably a site having a structure that physically or chemically interacts with a predetermined site or a functional group of the test substance.

The response site may have a structure that responds to the interaction between the recognition site and the test substance, examples thereof include a structure that emits light or develops color in response to the interaction

between the recognition site and the test substance, and from the viewpoint that a test substance can be detected with higher sensitivity, the response site preferably has a light-emitting structure.

Here, "light emitting" refers to generation of fluorescence or phosphorescence. From the viewpoint of improving the detection sensitivity of a test substance (preferably an endotoxin) by obtaining a large amount of light, generation of fluorescence is more preferable. The response site preferably includes a structure having a light-emitting structure, the light-emitting structure is preferably a structure having a conjugated multiple bond, and a part of the structure having a conjugated multiple bond is preferably cyclic. The site of the conjugated multiple bond may be a heterocyclic compound containing an atom (heteroatom) other than a carbon atom.

The response site and the recognition site may be directly bonded to each other or may be bonded to each other via a spacer described below, but from the viewpoint that a test substance can be detected with higher sensitivity, the specific compound preferably has a structure in which the response site and the recognition site are linked to each other via the spacer.

From the viewpoint that a test substance can be detected with high sensitivity, the specific compound is preferably a compound represented by the following Formula (1).

A-L-Y        Formula (1)

In Formula (1), A represents a response site which is a group containing a conjugated multiple bond, L represents a single bond or a spacer having a chain length of from 1 to 10 atoms, and Y represents a recognition site which is a group interacting with a test substance.

[Recognition Site: Y]

Y represents a recognition site which is a group interacting with a test substance.

The recognition site is preferably a group that physically or chemically interacts with a predetermined site or a functional group of the test substance, and is more preferably a group that specifically interacts with a specific site of the test substance, and in a case in which the recognition site recognizes the test substance, the group has a structure capable of transmitting information (hereinafter, referred to as "recognition information") to the response site.

The recognition information described here indicates, for example, a change in coordination state of electrons inside the chemical structure of the recognition site.

In a case in which the recognition site recognizes a specific site of the test substance, the coordination state of electrons changes in the chemical structure. This change in the coordination state of the electrons is transmitted as the recognition information to the response site described below. In the response site to which the recognition information has been transmitted, the coordination state of electrons changes, so that the light emission intensity or the color development intensity of the response site is changed (increased or attenuated), thereby visualizing that the recognition site recognizes the test substance (preferably the specific site of the test substance).

In the disclosure, in a case in which the recognition site and the test substance interact with each other, it can be estimated that the recognition site has recognized the test substance.

Examples of the mode of the interaction between the recognition site and the test substance include electrostatic force, intermolecular force, covalent bond, and hydrogen bond.

Y is preferably a group containing a metal coordinating group, an acid group, or a base group, and more preferably a metal coordinating group or an acid group.

The metal coordinating group may be a group that directly coordinates to at least one metal or a group that promotes coordination to a metal. Examples of the metal include metal ions (cations) ($Li^+$, $Na^+$, $K^+$, $Mg^{2+}$, $Ag^+$, $Ni^{2+}$, $Co^{2+}$, $Zn^{2+}$, or the like) of an alkali metal, an alkaline earth metal, a transition metal, and the like, and among these, a transition metal is preferable, a divalent transition metal is more preferable, a copper ion ($Cu^{2+}$), a nickel ion ($Ni^{2+}$), or a cobalt ion ($Co^{2+}$) is still more preferable, and $Cu^{2+}$ is particularly preferable from the viewpoint that a test substance can be detected with high sensitivity.

Examples of the metal coordinating group include groups including an aromatic heterocyclic group containing at least one atom selected from the group consisting of a nitrogen atom, a sulfur atom, and an oxygen atom, a hydroxyl group, a carboxy group, and a crown ether group.

Examples of the aromatic heterocyclic group include heterocyclic groups containing at least one atom selected from the group consisting of a nitrogen atom, a sulfur atom, and an oxygen atom. From the viewpoint that a test substance can be detected with high sensitivity, among them, the aromatic heterocyclic group is preferably a heterocyclic group containing at least one atom selected from the group consisting of a nitrogen atom and an oxygen atom, more preferably a heterocyclic group containing a nitrogen atom, still more preferably a 5- or 6-membered heterocyclic ring containing a nitrogen atom, and particularly preferably a 5-membered heterocyclic ring containing a nitrogen atom.

Examples of the heterocyclic ring containing a nitrogen atom include a pyrrole ring, a pyridine ring, a pyrazine

ring, and a pyrimidine ring.

The heterocyclic ring may be unsubstituted or may have a substituent, but is preferably a heterocyclic ring having a substituent from the viewpoint that a test substance can be detected with high sensitivity.

Examples of the substituent include an alkyl group and an aryl group, and among these, an alkyl group is preferable, and an alkyl group having from 1 to 4 carbon atoms is more preferable.

From the viewpoint that a test substance can be detected with high sensitivity, among heterocyclic rings having a substituent, a nitrogen atom-containing heterocyclic ring having an alkyl group is preferable, a 5- or 6-membered nitrogen atom-containing heterocyclic ring having an alkyl group having from 1 to 4 carbon atoms is more preferable, a 5-membered nitrogen atom-containing heterocyclic ring having an alkyl group having from 1 to 4 carbon atoms is still more preferable, and a pyridyl group having an alkyl group having from 1 to 4 carbon atoms is particularly preferable.

In a case in which the metal coordinating group contains an aromatic heterocyclic ring, from the viewpoint that a test substance can be detected with high sensitivity, the group containing an aromatic heterocyclic ring is preferably a substituted amino group substituted with a heterocyclic group, more preferably a substituted amino group substituted with a heterocyclic ring containing an alkyl group having from 1 to 4 carbon atoms (preferably a 5- or 6-membered nitrogen atom-containing heterocyclic ring having an alkyl group having from 1 to 4 carbon atoms, more preferably a 5-membered nitrogen atom-containing heterocyclic ring having an alkyl group having from 1 to 4 carbon atoms, and still more preferably a pyridyl group having an alkyl group having from 1 to 4 carbon atoms), and still more preferably a disubstituted amino group substituted with a heterocyclic ring containing an alkyl group having from 1 to 4 carbon atoms (preferably a 5- or 6-membered nitrogen atom-containing heterocyclic ring having an alkyl group having from 1 to 4 carbon atoms, more preferably a 5-membered nitrogen atom-containing heterocyclic ring having an alkyl group having from 1 to 4 carbon atoms, and still more preferably a pyridyl group having an alkyl group having from 1 to 4 carbon atoms).

In a case in which the metal coordinating group at the recognition site (preferably Y) is an aromatic heterocyclic group, it is preferable to add a metal ion capable of forming a complex that physically or chemically interacts with the functional group constituting the recognition site and a predetermined site or a functional group of the test substance (preferably an endotoxin). In a case in which the response site described below includes a structure capable of emitting fluorescence, for example, in a case in which the mechanism of light emission and quenching of the response site depends on Photo-induced Electron Transfer (PET), it is preferable to coordinate a transition metal ion (for example, a copper ion($Cu^{2+}$), a nickel ion ($Ni^{2+}$), or a cobalt ion ($Co^{2+}$)) whose d orbital is not closed as the metal ion to the recognition site. By coordinating a specific metal ion, the fluorescence of the response site is quenched in a case in which a test substance (preferably an endotoxin) is not present, so that the detection sensitivity can be enhanced together with an S/N ratio.

Since the amount of fluorescence emission is significantly increased in the case of coordination to the metal ion, a test substance (preferably an endotoxin) may be detectable as a decrease in the amount of luminescence. Examples of such a metal ion include metal ions in the above-described metal coordinating group, and examples thereof include a zinc ion ($Zn^{2+}$) and a cadmium ion ($Cd^{2+}$).

Examples of the acid group include a group selected from a sulfo group, a carboxy group, a phosphoric acid group, a boronic acid group, a phenol group, a salt thereof, and a desalted structure thereof, and among these, and a group selected from a carboxy group, a boronic acid, a salt thereof, and a desalted structure thereof is preferable.

Examples of the group containing the acid group include an alkyl group containing the acid group, an aryl group containing the acid group, and amino group containing the acid group. Among them, an aryl group containing the acid group or an amino group containing the acid group is preferable.

Examples of the substituent other than the acid group include an alkyl group and an aryl group, and among these, an alkyl group is preferable, and an alkyl group having from 1 to 4 carbon atoms is more preferable.

From the viewpoint that a test substance can be detected with high sensitivity, the group containing an acid group is preferably a phenyl group containing an acid group or an amino group containing an acid group, preferably a phenyl group containing an acid group or an amino group substituted with an alkyl group containing an acid group and having from 1 to 4 carbon atoms, more preferably a phenyl group containing boronic acid or an amino group substituted with an alkyl group containing a carboxy group and having from 1 to 4 carbon atoms, still more preferably a phenyl group containing boronic acid or a disubstituted amino group substituted with an alkyl group containing a carboxy group and having from 1 to 4 carbon atoms, and particularly preferably a phenyl group containing boronic acid or a disubstituted amino group substituted with an alkyl group containing a carboxy group and having 1 or 2 carbon atoms.

Examples of the disubstituted amino group substituted with an alkyl group containing a carboxy group and having 1 or 2 carbon atoms include monovalent groups obtained by removing a hydrogen atom from iminodiacetic acid.

Examples of the base group include an amino group and an aromatic ring containing a nitrogen atom. From the viewpoint that a test substance can be detected with high sensitivity, the base group is preferably an amino group or a guanidino group.

The metal coordinating group or the group containing an acid group is preferably a group represented by the following Formula (Y1) or Formula (Y2).

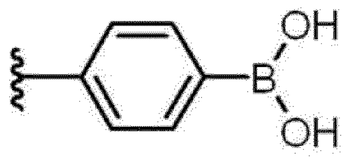

Formula (Y1)

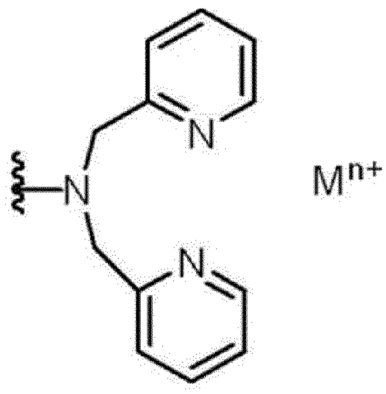

Formula (Y2)

In Formulae (Y1) and (Y2), each independently, a wavy line represents a linking site with L in Formula (1), $M^{n+}$ represents a metal ion, and n represents a natural number. n is preferably an integer from 1 to 3.
The metal ion has the same meaning as the metal ion in the above-described metal coordinating group, and a preferred aspect is also the same.
The group interacting with a test substance: Y is a site that interacts with a test substance, and can be set, if appropriate, according to the type of the test substance described below.

[A]

A represents a response site which is a group containing a conjugated multiple bond. The group containing a conjugated multiple bond may be linear or may have a ring structure. The site of the conjugated multiple bond may contain a heteroatom.
Examples of the group containing a conjugated multiple bond include a methine group (-C=C-), an aromatic group, an azo group, and a group obtained by combining these groups.
A is not particularly limited, and may be a group composed only of a conjugated multiple bond, but may have a substituent that does not form a conjugated bond, such as an alkyl group, if appropriate. For example, in a case in which an alkyl group is introduced as a substituent to A, compatibility between the specific compound and micelles, vesicles, or the like (that is, a property of forming a surface layer of a molecular assembly such as micelle or vesicle together with a surfactant) is improved, so that optical characteristics are improved, and the test substance can be measured to a lower concentration.
From the viewpoint that a test substance can be detected with high sensitivity, the group containing a conjugated multiple bond is preferably a group containing at least one of an aromatic group or an azo group.
The aromatic group may be an aromatic hydrocarbon ring or an aromatic heterocyclic ring. The ring structure may be a monocyclic ring or a polycyclic ring, and the polycyclic ring may be a fused ring. The aromatic group may be unsubstituted or may have a substituent, but is preferably unsubstituted from the viewpoint that a test substance can be detected with high sensitivity.
The aromatic heterocyclic ring may be a monocyclic ring, or may be a fused heterocyclic ring in which bicyclic or more heterocyclic rings are fused or a heterocyclic ring and an aromatic ring are fused, but is preferably a fused heterocyclic ring in which a heterocyclic ring and an aromatic ring are fused.
The number of members of the heterocyclic ring is not particularly limited, but is preferably from a 5-membered ring to an 8-membered ring, and more preferably a 5-membered ring or a 6-membered ring.
The heterocyclic ring is preferably a heterocyclic ring having at least one of at least one heteroatom selected from the group consisting of a nitrogen atom, an oxygen atom, and a sulfur atom, more preferably a heterocyclic ring having at least one heteroatom selected from the group consisting of a nitrogen atom and an oxygen atom, still more preferably a heterocyclic ring containing a nitrogen atom or an oxygen atom, and particularly preferably a heterocyclic ring containing a nitrogen atom.
Examples of the heterocyclic ring include a coumarin ring, a pyridine ring, a pyrimidine ring, a thiophene ring, a furan ring, and a pyrrole ring.
The heterocyclic ring may be unsubstituted or have a substituent. The substituent is not particularly limited, and examples thereof include a hydroxy group, a carboxy group, an alkoxy group, an aryloxy group, a halogen atom, an alkyl group, a halogenated alkyl group, an aryl group, an alkyloxycarbonyl group, an alkylcarbonyloxy group, an

alkoxycarbonyl group, an aryloxycarbonyl group, an alkylthio group, an arylthio group, a dialkylamino group, an alkylarylamino group, a diarylamino group, and a group obtained by combining two or more the these. Among them, the substituent is preferably at least one group selected from the group consisting of a hydroxy group and a carboxy group.

From the viewpoint that a test substance can be detected with high sensitivity, the heterocyclic ring is preferably a heterocyclic ring having a substituent, more preferably a coumarin ring having a substituent, still more preferably a coumarin ring substituted with at least one group selected from the group consisting of a hydroxy group and a carboxy group, and particularly preferably a coumarin ring substituted with a hydroxy group and a carboxy group.

The aromatic ring may be a monocyclic ring, or may be a fused aromatic ring in which bicyclic or more aromatic rings are fused, but is preferably a fused aromatic ring from the viewpoint that a test substance can be detected with high sensitivity. The fused aromatic ring is preferably a fused aromatic ring in which bicyclic to octacyclic aromatic rings are fused, more preferably a fused aromatic ring in which bicyclic to hexacyclic aromatic rings are fused, and still more preferably a fused aromatic ring in which bicyclic to tetracyclic aromatic rings are fused.

Examples of the fused aromatic ring include a naphthalene ring, an anthracene ring, a phenalene ring, a phenanthrene ring, a pyrene ring, a triphenylene ring, a tetracene ring, a quinoline ring, a chrysene ring, and a picene ring.

In a case in which A is an aromatic group, from the viewpoint that a test substance can be detected with high sensitivity, a heterocyclic ring or a fused aromatic ring is preferable, a coumarin ring, a pyrene ring, or an anthracene ring is more preferable, and a pyrene ring is particularly preferable.

In a case in which the aromatic ring is a monocyclic ring, the aromatic ring is preferably a benzene ring.

In a case in which the group containing an aromatic group contains a monocyclic aromatic ring, the group may contain one aromatic ring or two or more aromatic rings.

Examples of the group containing a monocyclic aromatic ring include a phenyl group, a biphenyl group, and a stilbene group, and a biphenyl group is preferable from the viewpoint that a test substance can be detected with high sensitivity.

The group containing an aromatic group is preferably a group represented by the following Formula ($A^{aro}$) from the viewpoint that a test substance can be detected with high sensitivity.

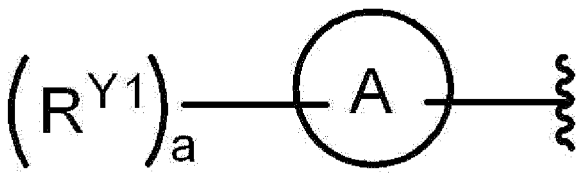

($A^{aro}$)

In Formula $A^{aro}$, A represents an aromatic ring, $R^{Y1}$ each independently represents a monovalent substituent, a represents an integer from 0 to 5, and a wavy line represents a binding site with L in Formula (1).

Examples of the aromatic ring include the aromatic ring in A described above, and among them, a benzene ring, a coumarin ring, a pyrene ring, or an anthracene ring is preferable.

Examples of $R^{Y1}$ each independently include the substituents in the aromatic hydrocarbon group described above. The substituent may be bonded to ring A via an amide bond or an ester bond.

The substituent is preferably an alkyl group, a hydroxy group, or a carboxy group. The alkyl group is preferably an alkyl group having from 1 to 15 carbon atoms, more preferably an alkyl group having from 2 to 12 carbon atoms, and still more preferably an alkyl group having from 5 to 10 carbon atoms.

In Formula $A^{aro}$, in a case in which a is an integer from 1 to 5, it is preferable that at least one $R^{Y1}$ is bonded to the ring A via an amide bond, and it is more preferable that at least one $R^{Y1}$ is an alkyl group having from 1 to 15 carbon atoms (preferably having from 2 to 12 carbon atoms, and more preferably having from 5 to 10 carbon atoms) bonded to the ring A via an amide bond.

a is preferably an integer from 0 to 3, and more preferably an integer from 0 to 2.

In a case in which the ring A is a coumarin ring, A is preferably represented by the following Formula $A^{cou}$.

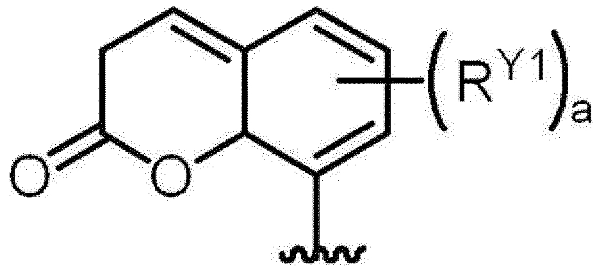

($A^{cou}$)

In Formula $A^{cou}$, $R^{Y1}$ each independently represents a monovalent substituent, a represents an integer of 1 or 2, and a wavy line represents a binding site with L in Formula (1).

The substituent in $R^{Y1}$ has the same meaning as the substituent in $A^{aro}$, and a preferred aspect is also the same.

A group including an azo (-N=N-) structure (hereinafter, also referred to as "azo group") may include one azo structure or two or more azo structures, but from the viewpoint that a test substance can be detected with high sensitivity, the group is preferably a group including one azo structure, and more preferably a group obtained by combining an azo group and an aromatic group. In the group obtained by combining an azo group and an aromatic group, the aromatic group may be an aromatic hydrocarbon ring or an aromatic heterocyclic ring, but is preferably an aromatic hydrocarbon group from the viewpoint of excellent color developability.

The aromatic ring of the aromatic hydrocarbon group may be a monocyclic ring, or may be a fused aromatic ring in which bicyclic or more aromatic rings are fused. Examples of the fused aromatic ring include a naphthalene ring, an anthracene ring, and a pyrene ring.

In a case in which the aromatic ring is a monocyclic ring, the aromatic ring is preferably a benzene ring.

The aromatic hydrocarbon group may be unsubstituted or may have a substituent. The substituent is not particularly limited, and examples thereof include a hydroxy group, a carboxy group, an alkoxy group, an aryloxy group, a halogen atom, an alkyl group, a halogenated alkyl group, an aryl group, an alkyloxycarbonyl group, an alkylcarbonyloxy group, an alkoxycarbonyl group, an aryloxycarbonyl group, an alkylthio group, an arylthio group, a dialkylamino group, an alkylarylamino group, a diarylamino group, and a group obtained by combining two or more the these.

The group including an azo (-N=N-) structure is a preferably a group in which an aromatic hydrocarbon group is bonded to an azo structure, and more preferably a group represented by the following Formula ($A^{azo}$) from the viewpoint that a test substance can be detected with high sensitivity.

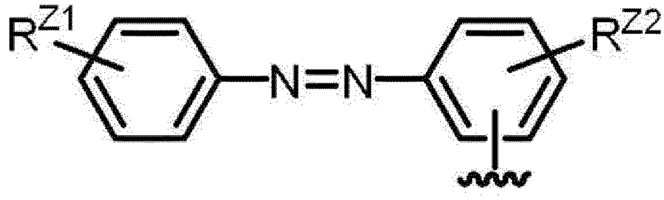

($A^{azo}$)

In Formula ($A^{azo}$), each of $R^{Z1}$ and $R^{Z2}$ independently represents a substituent, and a wavy line represents a binding site with L in Formula (1).

Examples of the substituent include the substituents in the aromatic hydrocarbon group described above. The substituent may be bonded to an azobenzene structure via an amide bond or an ester bond.

The substituent is preferably an alkyl group, a hydroxy group, or a carboxy group. The alkyl group is preferably

an alkyl group having from 1 to 15 carbon atoms, more preferably an alkyl group having from 2 to 12 carbon atoms, and still more preferably an alkyl group having from 5 to 10 carbon atoms.

$R^{Z1}$ and $R^{Z2}$ may have the same structure or may be different from each other, but $R^{Z1}$ and $R^{Z2}$ are preferably different from each other from the viewpoint of micelle formation.

$R^{Z1}$ and $R^{Z2}$ may be bonded to an azo group at any position of an ortho position, a meta position, and a para position. Among them, the substituents in $R^{Z1}$ and $R^{Z2}$ are preferably bonded to the azo group at the para position.

The binding site with L may be bonded to the azo group at any position of an ortho position, a meta position, and a para position. Among them, the binding site with L is preferably bonded to the azo group at the meta position.

Specific examples of A in Formula (1) include coumarin represented by the following Formula (5A) (hereinafter, referred to as "C"), biphenyl represented by the following Formula (6A), pyrene represented by the following Formula (7A) (hereinafter, referred to as "Pr"), stilbene represented by the following Formula (8A), anthracene represented by the following Formula (9A), azobenzene represented by the following Formula (10A), derivatives thereof, an organic electroluminescence dye, and a fluorescent protein. The disclosure is not limited to the following specific examples.

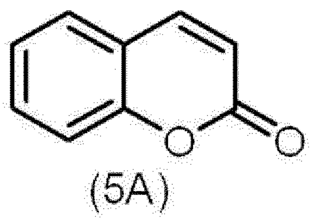

(5A)

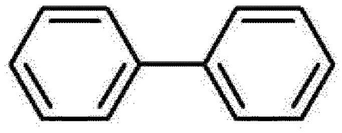

(6A)

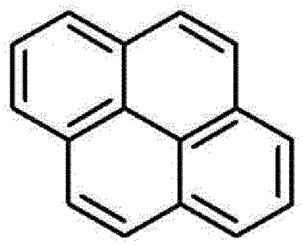

(7A)

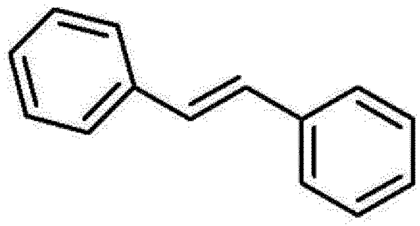

(8A)

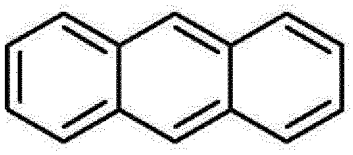

(9A)

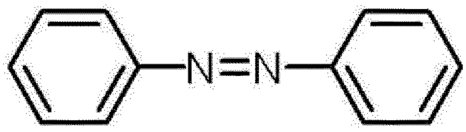

(10A)

Examples of the coumarin derivative that can be used as a response site include coumarin 1, coumarin 6, coumarin 7, coumarin 30, 7-hydroxycoumarin-3-carboxylic acid, and 8-(2,2'-dipicolylaminomethyl)-7-hydroxycoumarin-3-carboxylic acid, and 7-hydroxycoumarin-3-carboxylic acid is most preferable.

Examples of the biphenyl derivative that can be used as a response site include 4-biphenylcarboxylic acid, 2-(4'-t-butylphenyl)-5-(4'-biphenyl)1,3,4-oxadiazole, 2,5-bis-(2-(4-biphenyl)ethenyl)pyrazine, and 4,4'-bis(2,2-diphenylvinyl)biphenyl, and 4-biphenylcarboxylic acid is most preferable from the viewpoint of detection sensitivity.

Examples of the pyrene derivative that can be used as the response site include alkynylpyrene, and the pyrene represented by the above Formula (7A) is most preferable from the viewpoint of detection sensitivity.

Examples of the stilbene derivative that can be used as a response site include 1,1,4,4-tetraphenyl-1,3-butadiene, 4,4'-bis(2,2-diphenylvinyl)biphenyl, and cyanostilbene, and the stilbene represented by the above Formula (8A) is most preferable from the viewpoint of detection sensitivity.

Examples of the anthracene derivative that can be used as a response site include 10-[2-(9anthracenyl)ethyl] phenoxazine, 10-[2-(9-anthracenyl)ethyl]phenothiazine, and 2-(9-anthracenyl)ethyldiphenylamine, and the anthracene

represented by the above Formula (9A) is most preferable from the viewpoint of detection sensitivity.

Examples of the azobenzene derivative that can be used as a response site include derivatives having the group represented by the above Formula ($A^{azo}$), and a preferred aspect is also the same.

Examples of the fluorescent protein that can be used as the response site include Green Fluorescent Protein (GFP), Blue Fluorescent Protein (BFP), Cyan Fluorescent Protein (CFP), Enhanced Green Fluorescent Protein (EGFP), Enhanced Yellow Fluorescent Protein (EYFP), and Photoactivatable Green Fluorescent Protein (PA-GFP), and GFP is most preferable.

In a case in which the response site includes a structure that emits fluorescence, the structure that emits fluorescence often exhibits hydrophobicity, and the recognition site often exhibits hydrophilicity. Therefore, for example, by making the type of the spacer appropriate and imparting sufficient hydrophilicity to the fluorescent substance described below, the fluorescent substance has water solubility, and it is not necessary to dissolve the fluorescent substance in, for example, an organic solvent or the like for use, and the detection accuracy can be enhanced.

As a method of making the type of spacer appropriate, for example, a method of adjusting the number of carbon atoms of the chain length of the spacer: L described below, introducing a hydrophilic group into a side chain, or introducing an atom such as nitrogen into a chain is considered. Similar adjustment can be performed on the response site or the recognition site.

In a case in which the response site includes a structure that emits fluorescence, the specific compound has a schematic configuration as illustrated in the schematic view of Fig. 1. For example, a recognition site 40 that interacts with a test substance (preferably an endotoxin) 50 and a fluorescent site 20 that emits light are linked to each other via a spacer 30. The metal ion may be coordinated to the recognition site 40 depending on the chemical structure.

In a case in which the response site includes a structure that emits fluorescence, specific examples of the specific compound (fluorescent substance) include dpa-HCC represented by the above Formula (1) in which a dipicolylamino group to which the metal ion represented by the above Formula (Y2) is coordinated and 7-hydroxycoumarin-3-carboxylic acid, which is a derivative of the coumarin represented by the above Formula (5A) are bonded to each other via a spacer having a chain length of one carbon atom.

Specific examples of A into which an alkyl chain or the like is introduced include the following.

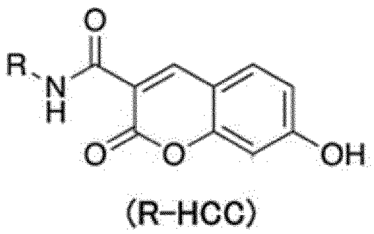

(R-HCC)

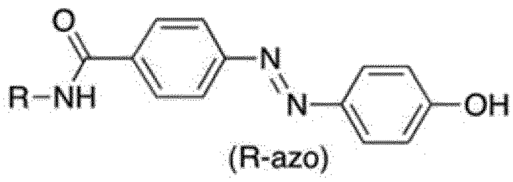

(R-azo)

R-HCC (hydroxycoumarin carboxylic acid alkyl amide) is obtained by introducing an alkyl chain into coumarin via an amide bond. Similarly, R-azo (hydroxyazobenzene carboxylic acid alkyl amide) is obtained by introducing an alkyl chain into 4-hydroxyazobenzene via an amide bond.

R in the above R-HCC and R-azo represents an alkyl group (preferably an alkyl group having from 1 to 15 carbon atoms, more preferably an alkyl group having from 2 to 12 carbon atoms, and still more preferably an alkyl group having from 5 to 10 carbon atoms).

Other specific examples of the specific compound (fluorescent substance) include C4-APB in which phenylboronic acid represented by the above Formula (Y1) (hereinafter, referred to as "PB") and the pyrene ring represented by the above Formula (7A) are linked to each other via an amide bond (-C(=O)NH-) as the spacer 30.

[L]

L represents a single bond or a spacer having a chain length of from 1 to 10 atoms. In a case in which L is a single bond, since L is a linking group, in Formula (1), it represents that A and Y are directly bonded.

The spacer mediates the binding between the response site and the recognition site. From the viewpoint of efficiently transferring the recognition information from the recognition site to the response site, the spacer preferably has a chain length of from 1 to 10 atoms.

In the present specification, the chain length is a value obtained by counting the number of atoms from the response site to the recognition site via the spacer so as to be minimum, and the number of atoms included in the response site and the recognition site is not included in the length of the chain length.

For example, the chain length in the following compound dpa-C4Py is 4. The chain length in the following compound C4-APB is 6.

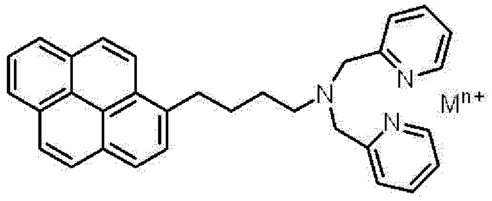

(dpa-C4Py)

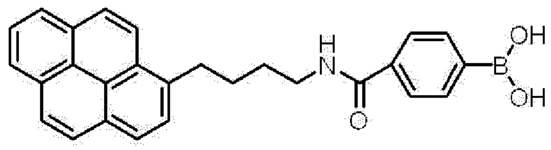

(C4-APB)

The chain constituting the chain length is preferably a chain containing at least one atom selected from the group consisting of a carbon atom, an oxygen atom, a nitrogen atom, and a sulfur atom, more preferably a chain containing at least one atom selected from the group consisting of a carbon atom, an oxygen atom, and a sulfur atom, still more preferably a chain containing at least one selected from the group consisting of an alkyl chain, an ether bond (-O-), an amide bond (-C(=O)NH-), and an ester bond (-C(=O)O-), and particularly preferably a chain containing at least one selected from the group consisting of an alkyl chain and an amide bond.

L may be linear (straight chain) or branched (branched chain), but is preferably a linear saturated alkyl chain from the viewpoint that a test substance can be detected with high sensitivity.

In order to have a plurality of recognition sites in the specific compound, L may be bonded to the recognition site as a branched chain. L may be a branched chain, and a hydrophilic group may be introduced into a side chain.

Examples of the hydrophilic group include a hydroxy group, a carboxy group, and an amino group.

From the viewpoint that a test substance can be detected with high sensitivity, L is preferably a chain having a chain length of from 1 to 10 atoms and containing at least one selected from the group consisting of an alkyl chain and an amide bond, more preferably an alkyl chain having a chain length of from 1 to 8 atoms, still more preferably a linear alkyl chain having a chain length of from 1 to 8 atoms, and particularly preferably a linear alkyl chain having a chain length of from 1 to 6 atoms.

Specific examples of the specific compound (preferably the compound represented by Formula (1)) include the following exemplary compounds. The disclosure is not limited to the following specific examples. Hereinafter, the following exemplary compounds may be represented by symbols in parentheses. In the exemplary compounds, $M^{n+}$ represents a metal ion and has the same meaning as $M^{n+}$ described above.

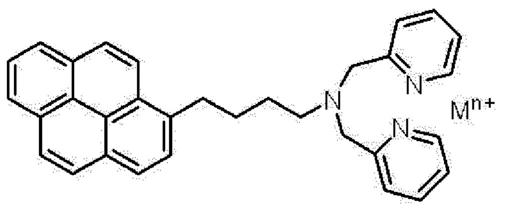

(dpa-C4Py)

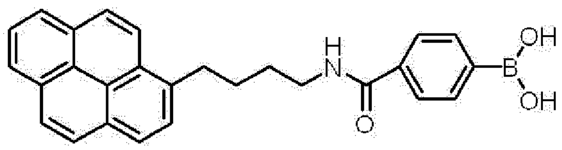

(C4-APB)

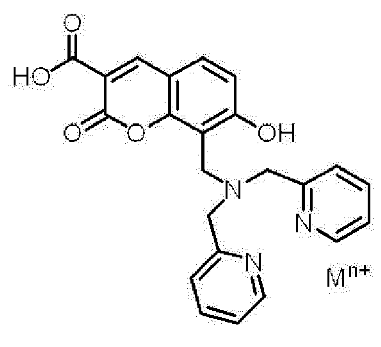

(dpa-HCC)

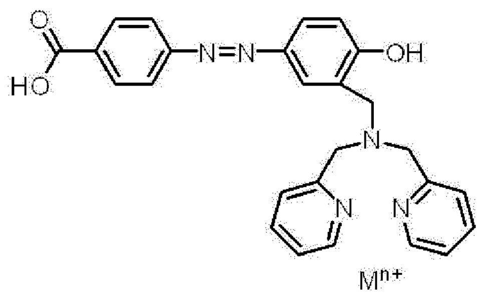

(dpa-azo)

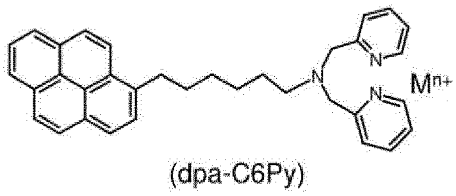

(dpa-C6Py)

In the exemplary compounds, dpa-C4Py is N,N'-(dipicolyl)-4-(1-pyrenyl)butylamine, C4-APB is phenylboronic acid-4-(1-pyrenyl)butyramide, dpa-HCC is dipicolylamine-hydroxycoumarin carboxylic acid, dpa-azo is dipicolylamine-hydroxyazobenzene, and dpa-C6Py is N,N'-(dipicolyl)-4-(1-pyrenyl)hexylamine.

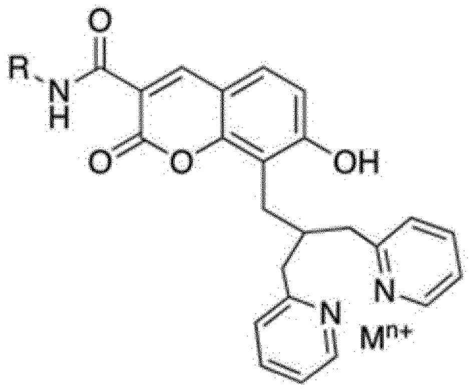

(dpa-R-HCC)

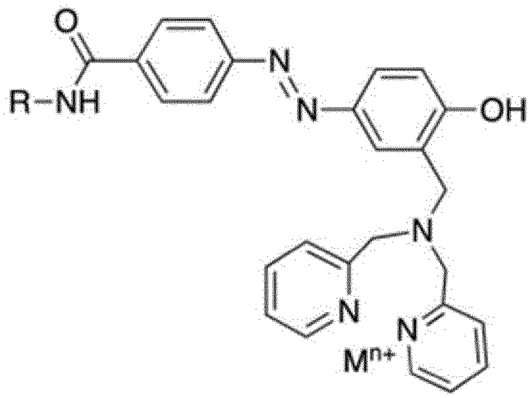

(dpa-R-azo)

dpa-R-HCC is dipicolylamine-hydroxycoumarin carboxylic acid alkyl amide, and dpa-R-azo is dipicolylamine-hydroxyazobenzene carboxylic acid alkyl amide. R in the above dpa-R-HCC and dpa-R-azo represents an alkyl group (preferably an alkyl group having from 1 to 15 carbon atoms, more preferably an alkyl group having from 2 to 12 carbon atoms, and still more preferably an alkyl group having from 5 to 10 carbon atoms).

From the viewpoint of detecting a test substance with high sensitivity, the specific compound is preferably a compound capable of forming a molecular assembly with a surfactant described below in an aqueous solution, and more preferably a compound capable of forming micelles or vesicles with a surfactant described below in an aqueous solution.

The specific compound may be used singly, or in combination of two or more kinds thereof.

In the detection method according to the disclosure, a plurality of fluorescent substances having a recognition site that recognizes different specific sites of a test substance may be used. As a result, multipoint recognition of a test substance (for example, an endotoxin) becomes possible, and the specificity and the detection sensitivity further increase. For example, a mixture of dpa-HCC and C4-APB is added as a fluorescent reagent to a specimen, and the fluorescence intensity can be detected at two wavelengths. Even in the case of using a specific compound having a plurality of recognition sites or a specific compound having a plurality of response sites, a similar effect can be obtained. In this case, as the spacer, one having a side chain may be used, or a plurality of spacers may be used.

<Surfactant>

In the detection method according to the disclosure, the type of surfactant is not limited. The surfactant in the disclosure includes a known surfactant.

Examples of the surfactant include a cationic surfactant, an anionic surfactant, an amphoteric surfactant, and a non-ionic surfactant.

From the viewpoint that a test substance can be detected with high sensitivity, the surfactant is preferably a cationic surfactant, an amphoteric surfactant, or a non-ionic surfactant, and more preferably a cationic surfactant.

Examples of the cationic surfactant include surfactants having a nitrogen atom, and specific examples thereof include alkyltrimethylammonium salts (for example, stearyltrimethylammonium chloride and lauryltrimethylammonium chloride), dialkyldimethylammonium salts (for example, distearyldimethylammonium chloride), alkylpyridinium salts (for example, poly(N,N-dimethyl-3,5-methylenepiperidinium) chloride and cetylpyridinium chloride), alkyl quaternary ammonium salts, alkyldimethylbenzylammonium salts, alkylisoquinolinium salts, dialkylmorphonium salts, polyoxyethylenealkylamines, alkylamine salts, polyamine fatty acid derivatives, amyl alcohol fatty acid derivatives, benzalkonium chloride,

and benzethonium chloride.

Among them, from the viewpoint that a test substance can be detected with high sensitivity, the cationic surfactant is preferably cetyltrimethylammonium bromide (CTAB, HLB: 10), didodecyldimethylammonium bromide (DDAB), or dodecyltrimethylammonium chloride (DTAC), and more preferably cetyltrimethylammonium bromide (CTAB, HLB: 10) or didodecyldimethylammonium bromide (DDAB).

The cationic surfactant may be used singly, or in combination of two or more kinds thereof.

Examples of the anionic surfactant include higher fatty acid salts (for example, sodium cholate, potassium stearate, and potassium behenate), alkyl ether carboxylates (for example, sodium polyoxyethylene lauryl ether carboxylate), N-acyl-L-glutamates (for example, N-stearoyl-L-glutamic acid monosodium salt), higher alkyl sulfuric acid ester salts (for example, sodium dodecyl sulfate (SDS), sodium lauryl sulfate, and potassium lauryl sulfate), polyoxyethylene lauryl sulfate triethanolamine, alkyl ether sulfuric acid ester salts (for example, sodium polyoxyethylene lauryl sulfate), N-acyl sarcosinates (for example, sodium lauroyl sarcosine), alkyl phosphates (for example, sodium stearyl phosphate), alkyl ether phosphates (for example, sodium polyoxyethylene oleyl ether phosphate and sodium polyoxyethylene stearyl ether phosphate), higher fatty acid ester sulfates (for example, hydrogenated coconut oil fatty acid glycerin sodium sulfate), and phosphatidylglycerols (dipalmitoylphosphatidylglycerol, dimyristoylphosphatidylglycerol, distearoylphosphatidylglycerol, dioleoylphosphatidylglycerol (DOPG), and the like).

Among them, from the viewpoint that a test substance can be detected with high sensitivity, the anionic surfactant is preferably sodium dodecyl sulfate (SDS, HLB: 40), dioleoylphosphatidylglycerol (DOPG), or sodium cholate.

The anionic surfactant may be used singly, or in combination of two or more kinds thereof.

Examples of the non-ionic surfactant include monofatty acid sorbitan (for example, polysorbate 20 (polyoxyethylene sorbitan monolaurate), polysorbate 80 (polyoxyethylene sorbitan oleate)), polyoxyethylene-hydrogenated castor oil, polyoxyethylene alkyl ether, and polyoxyethylene alkyl phenyl ether.

Among them, from the viewpoint that a test substance can be detected with high sensitivity, the non-ionic surfactant is preferably polysorbate (PS), octoxynol, (polyoxyethylene)n ethyl ether (n = 10 (HLB: 16)), (polyoxyethylene)n-p-t-octylphenyl ether (n = 9 to 10 (HLB: 14)) or (polyoxyethylene)n sorbitan monostearate (n = 20 (HLB: 15)), and more preferably (polyoxyethylene)n ethyl ether (n = 10 (HLB: 16)), (polyoxyethylene)n-p-t-octylphenyl ether (n = 9 to 10 (HLB: 14)), or (polyoxyethylene)n sorbitan monostearate (n = 20 (HLB: 15)).

The non-ionic surfactant may be used singly, or in combination of two or more kinds thereof.

Examples of the amphoteric surfactant include carboxybetaine, aminocarboxylic acid, sulfobetaine, aminosulfate ester, imidazoline, and a phospholipid (lecithin).

Examples of the phospholipid include phospholipids such as diphthaloyl-sn-glycero-3-phosphocholine (DPhPC), 1,2-dipalmitoyl-sn-glycero-3-phosphocholine (DPPC), 1,2-dimyristoyl-sn-glycero-3-phosphocholine (DMPC), diphytanoylphosphatidylethanolamine (DPhPE), 1,2-distearoyl-sn-glycero-3-phosphocholine (DSPC), 1,2-dipalmitoyl-sn-glycero-3-phosphoethanolamine (DPPE), and 1,2-dioleoyl-sn-glycero-3-phosphoethanolamine (DOPE).

Among them, from the viewpoint that a test substance can be detected with high sensitivity, the amphoteric surfactant is preferably 1,2-dilinoleoyl-sn-glycero-3-phosphocholine (DOPC) or dodecyl-N-betaine, and more preferably 1,2-dilinoleoyl-sn-glycero-3-phosphocholine (DOPC).

The amphoteric surfactant may be used singly, or in combination of two or more kinds thereof.

The molecular weight of the surfactant is not limited. From the viewpoint of forming a molecular assembly, the molecular weight of the surfactant may be 1000 or less, from 150 to 1000, or from 200 to 500.

In the disclosure, the molecular weight of a surfactant having a molecular weight distribution is represented by a weight average molecular weight. In the disclosure, the weight average molecular weight is measured by gel permeation chromatography (GPC).

From the viewpoint that a test substance can be detected with high sensitivity, the HLB value of the surfactant is preferably from 6 to 40, more preferably from 7 to 30, and still more preferably from 7 to 20.

The value of hydrophilic-lipophilic balance (hereinafter, also referred to as "HLB value") HLB usually means the hydrophilic-hydrophobic balance used in the field of surfactants. The value of hydrophilic-lipophilic balance (hereinafter, also referred to as "HLB value") is calculated using Kawakami formula shown below. In the case of using a commercially available product as a glycerin fatty acid ester, commercially available catalog data is preferentially employed.

$$HLB = 7 + 11.7\log (Mw/Mo)$$

Mw represents the molecular weight of the hydrophilic group, and Mo represents the molecular weight of the hydrophobic group.

In the step A, it is presumed that in a case in which the concentration of the surfactant is equal to or less than the critical micelle concentration, micelles or forms close to micelles are partially formed, but from the viewpoint that a

test substance can be detected with higher sensitivity, the concentration of the surfactant at room temperature (25°C) with respect to a mixed liquid of the sample containing a test substance and water, the specific compound, and the surfactant is more preferably equal to or more than the critical micelle concentration. The concentration of the surfactant can also be set with reference to the detection result, if appropriate.

For example, measurement may be performed using a standard substance to set the concentration of the surfactant.

The critical micelle concentration can be measured by a dye solubilization method, and the critical micelle concentration means a concentration at which adsorption to a gas-liquid interface is saturated and micelle formation starts inside a solution. The content of the surfactant in the disclosure is preferably from 0.1 times to 100 times, more preferably 0.5 times to 100 times, and still more preferably 1 time to 50 times the critical micelle concentration.

The specific content of the surfactant is preferably from $1\times10^{-6}$% by mass to 1% by mass and more preferably from $1\times10^{-5}$% by mass to 0.1% by mass with respect to the total mass of the solution containing the test substance, the specific compound, and the surfactant.

The content ratio of the specific compound and the surfactant is preferably from 1 : 1 to 1 : 400 and more preferably from 1 : 10 to 1 : 200 on a mass basis.

[Test Substance]

Suitable examples of the test substance include a compound capable of forming a molecular assembly in an aqueous solution. Examples of the compound capable of forming a molecular assembly in an aqueous solution include phospholipids, glycolipids, fatty acids, surfactants other than the surfactant used in the detection method according to the disclosure described below (hereinafter, also referred to as "other surfactants"), and bacteria.

Examples of the other surfactants include perfluorooctanesulfonic acid (PFOS) and perfluorooctanoic acid (PFOA).

In a case in which the compound capable of forming a molecular assembly in an aqueous solution contains an alkali metal, a transition metal (Na, K, Mg, Ag, Ni, Co, Zn, or the like), and the like, the detection method according to the disclosure can also detect these metals.

In a case in which the test substance is a bacterium (preferably a gram-negative bacterium), the cell wall of the bacterium is broken down by the surfactant, and a phospholipid, a glycolipid, an endotoxin, and the like contained in the cell wall are incorporated into the molecular assembly, whereby detection becomes possible.

Among them, from the viewpoint that a test substance can be detected with high sensitivity, the test substance preferably contains a phospholipid, a glycolipid, or other surfactants containing phosphoric acid, more preferably contains at least one of a phospholipid or a glycolipid, and still more preferably contains an endotoxin.

The endotoxin has a structure in which a sugar chain as a hydrophilic site and an acyl acid as a hydrophobic site are bonded via two molecules of glucosamine. Phosphoric acid is bonded to each glucosamine in an endotoxin (Kenichi Tanamoto "Endotoxin and the Quality Control of Medicine" Bull. Natl. Inst. Health Sci., 126, 19-33 (2008)). In the endotoxin, a moiety in which two glucosamines having an acyl acid bonded thereto are bonded is referred to as lipid A, and most of the biological activity of the endotoxin is derived from this moiety.

Examples of the phospholipid include phosphatidylcholine.

Examples of the glycolipid include galactolipid.

Examples of the surfactant containing phosphoric acid include alkyl phosphates such as lauryl phosphate and polyoxyalkylene alkyl ether phosphate.

-Case in Which Test Substance Contains Glycolipid-

In a case in which the test substance is, for example, a glycolipid (preferably an endotoxin, more preferably a sugar moiety of an endotoxin), Y is preferably a group containing an acid group, more preferably a phenyl group containing an acid group, and still more preferably a phenyl group containing boronic acid.

A is preferably a fused aromatic ring in which bicyclic to tetracyclic aromatic rings are fused, more preferably a fused aromatic ring in which tricyclic or tetracyclic aromatic rings are fused, still more preferably a pyrene ring or a coumarin ring, and particularly preferably a pyrene ring.

L has the same meaning as L described above, and a preferred aspect is also the same.

In a case in which the test substance is an endotoxin (preferably, a sugar moiety of an endotoxin is recognized), from the viewpoint that a test substance can be detected with high sensitivity, the specific compound is preferably a compound represented by the following Formula 1A, C4-APB, dpa-R-HCC, or dpa-R-azo.

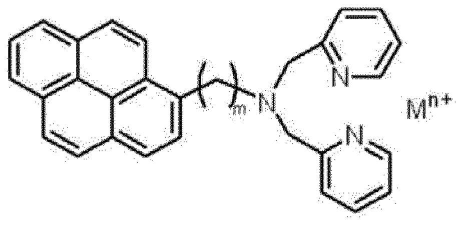

Formula (1A)

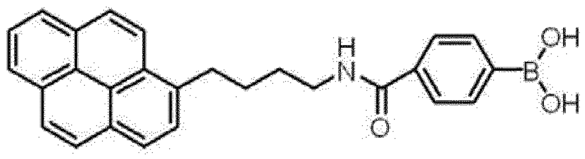

(C4-APB)

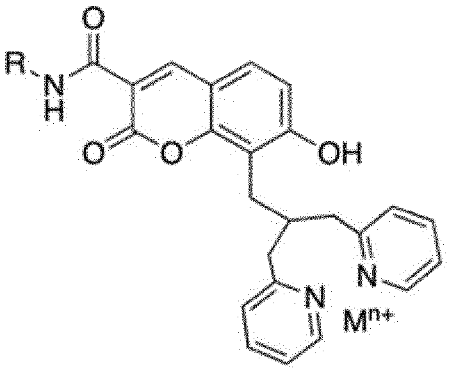

(dpa-R-HCC)

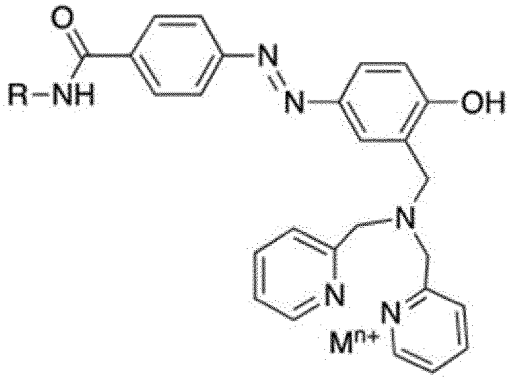

(dpa-R-azo)

In Formula (1A), m represents from 0 to 6, $M^{n+}$ represents a metal ion, and n represents a natural number. n is preferably an integer from 1 to 3.

The metal ion has the same meaning as the metal ion in the above-described metal coordinating group, and a preferred aspect is also the same. R in the above dpa-R-HCC and dpa-R-azo represents an alkyl group (preferably an alkyl group having from 1 to 15 carbon atoms, more preferably an alkyl group having from 2 to 12 carbon atoms, and still more preferably an alkyl group having from 5 to 10 carbon atoms).

-Case in Which Test Substance Contains Phosphoric Acid or Phospholipid-

In a case in which the test substance is, for example, phosphoric acid or a phospholipid, Y is preferably a metal coordinating group, preferably a substituted amino group substituted with a heterocyclic group, more preferably a substituted amino group substituted with a heterocyclic ring containing an alkyl group having from 1 to 4 carbon atoms (preferably a 5- or 6-membered nitrogen atom-containing heterocyclic ring having an alkyl group having from 1 to 4 carbon atoms, more preferably a 5-membered nitrogen atom-containing heterocyclic ring having an alkyl group having from 1 to 4 carbon atoms, and still more preferably a pyridyl group having an alkyl group having from 1 to 4 carbon atoms), and still more preferably a disubstituted amino group substituted with a heterocyclic ring containing an alkyl group having from 1 to 4 carbon atoms (preferably a 5- or 6-membered nitrogen atom-containing heterocyclic ring having an alkyl group having from 1 to 4 carbon atoms, more preferably a 5-membered nitrogen atom-containing heterocyclic ring having an alkyl group having from 1 to 4 carbon atoms, and still more preferably a pyridyl group having an alkyl group having from 1 to 4 carbon atoms).

A is preferably a group in which an aromatic hydrocarbon group is bonded to an azo structure, a heterocyclic ring, or a fused aromatic ring, and is preferably a group in which a phenyl group is bonded to an azo structure, a coumarin ring, a pyrene ring, or an anthracene ring.

L is more preferably a chain having a chain length of from 1 to 10 atoms and containing at least one selected from the group consisting of an alkyl chain and an amide bond, more preferably an alkyl chain having a chain length of from 1 to 8 atoms or a chain having a chain length of from 1 to 8 atoms and containing an amide bond, still more preferably a linear alkyl chain having a chain length of from 1 to 8 atoms or a chain having a chain length of from 1 to 6 atoms and containing an amide bond, and particularly preferably a linear alkyl chain having a chain length of from 1 to 6 atoms or a chain having a chain length of from 1 to 4 atoms and containing an amide bond.

-Case in Which Test Substance Contains Alkali Metal-

In a case in which the test substance contains, for example, an alkali metal, Y is preferably a metal coordinating group, and more preferably a group containing a crown ether group or the like.
L and A have the same meaning as L and A in the above Formula (1), and preferred aspects are also the same.

-Case in Which Test Substance Contains Transition Metal-

In a case in which the test substance contains, for example, a transition metal, Y is preferably a group containing an acid group, preferably a phenyl group containing an acid group or an amino group containing an acid group, preferably an amino group substituted with an alkyl group containing an acid group and having from 1 to 4 carbon atoms, more preferably an amino group substituted with an alkyl group containing a carboxy group and having from 1 to 4 carbon atoms, still more preferably a disubstituted amino group substituted with an alkyl group containing a carboxy group and having from 1 to 4 carbon atoms, and particularly preferably a disubstituted amino group substituted with an alkyl group containing a carboxy group and having 1 or 2 carbon atoms.
L and A have the same meaning as L and A in the above Formula (1), and preferred aspects are also the same.

<Sample>

The sample used in the step A contains the test substance and water.
Examples of the sample include samples derived from environments such as foods (fresh foods, processed foods, and the like), water (drinking water, groundwater, river water, seawater, domestic wastewater, industrial wastewater, and the like), and soil, body fluids such as blood, urine, saliva, and nasal secretions collected from humans or non-human animals, and tissue fluids.
Water contained in the sample may be water derived from the sample, or may be water separately added. The water to be separately added is not particularly limited, and may be distilled water, ion-exchanged water, deionized water, ultrafiltered water, pure water, or the like.
In the step A, components (other components) other than the sample, the specific compound, and the surfactant may be added. Examples of the other components include a buffer solution and a salt solution.
In the step A, the method of mixing the sample, the specific compound, and the surfactant is not particularly limited, and a known mixing method can be used.
The order of adding the specific compound and the surfactant to the sample is not particularly limited, and for example, the specific compound may be added after adding the surfactant to the sample, or the surfactant and the specific compound may be mixed to obtain a mixed liquid and then the mixed liquid may be added to the sample.

-Temperature-

In the step A, the temperature at which the specific compound and the surfactant are added to the sample is preferably from 15 to 35°C.

-pH-

In the step A, the pH of the mixed liquid of the sample, the specific compound, and the surfactant at 25°C is preferably from 5.0 to 9.0 and more preferably from 7.0 to 8.0.

<Step B>

The detection method according to the disclosure includes a step B of detecting a response at the response site arising from interaction between the recognition site and the test substance.
In the present specification, the response at the response site means a change in characteristics (examples thereof include changes in fluorescence, light absorption, an electrochemical method, zeta potential, light scattering, and visual observation, and are preferably changes in optical characteristics) at the response site arising from interaction between the test substance and the recognition site. Examples of the change in optical characteristics include a change in absorption wavelength, emission (fluorescence) wavelength or emission (fluorescence) intensity, and wavelength of excitation light.
Regarding the change in optical characteristics, the structure of a complex due to inclusion of a complex of a specific compound and a test substance in an association may be changed to cause a change in emission (fluorescence) wavelength or intensity to be observed.

An example of the change in optical characteristics at the response site arising from the interaction will be described with reference to dpa-HCC which is a specific compound.

7-Hydroxycoumarin-3-carboxylic acid which is a response site (fluorescent site) of dpa-HCC, alone shows a fluorescence intensity peak at 448 nm with respect to an excitation wavelength of, for example, 396 nm. In dpa-HCC in which a dipicolylamino group is bonded to the 7-hydroxycoumarin-3-carboxylic acid via a spacer ($-CH_2-$), a non-covalent electron pair of a branched nitrogen atom of the dipicolylamino group moves, as an electron donor unit, to the response site (fluorescent site) by Photo-induced Electron Transfer (PET), and fluorescence is partially suppressed. In a case in which a copper ion, a nickel ion, or a cobalt ion (hereinafter, referred to as "copper ion or the like") is coordinated to the dpa-HCC as a metal ion represented by the above Formula (Y2), fluorescence is attenuated by Ligand to Metal Charge Transfer (LMCT) in which electrons flow from 7-hydroxycoumarin-3-carboxylic acid as the response site (fluorescent site) to an empty d orbital such as a copper ion.

However, in a case in which the test substance is an endotoxin, in dpa-HCC, the attenuated fluorescence is restored. That is, it is considered that in a case in which the phosphoric acid group of the endotoxin is coordinated to the copper ion or the like coordinated to the dipicolylamino group, the chemical bond between the copper ion or the like and dpa-HCC is weakened, so that the attenuated fluorescence of 7-hydroxycoumarin-3-carboxylic acid is restored.

That is, the specific site recognized by the recognition site of dpa-HCC as the specific compound (fluorescent substance) is a phosphoric acid group of the endotoxin.

Other examples of the change in optical characteristics include C4-APB which is a specific compound. In the absence of the endotoxin, C4-APB is in a state in which fluorescence is attenuated by PET in which electrons flow into phenylboronic acid from a pyrene ring as the response site. On the other hand, in the presence of the endotoxin, C4-APB emits fluorescence.

That is, it is considered that the electron acceptability of phenylboronic acid is reduced by the interaction between phenylboronic acid and the hydroxyl group present in the sugar chain moiety of the endotoxin, and the fluorescence of pyrene which has been attenuated is restored.

That is, the specific site recognized by the recognition site of C4-APB as the specific compound (fluorescent substance) is a sugar chain moiety of the endotoxin.

The detection method of the response at the response site can be set according to the structure of the response site and the optical characteristics to be detected, if appropriate. As the detection apparatus, for example, an absorptiometer, a light emission detector, a fluorescence detector, or the like, which is known, can be used. Details of the detection apparatus will be described below.

<Other Steps>

The detection method according to the disclosure may further include steps (other steps) other than the step A and the step B.

Examples of the other steps include a sample pretreatment step (for example, a step of separating the sample from a substance other than the test substance, such as ion exchange), a sample concentration step (for example, a reverse osmosis treatment), and a vesicle formation step (for example, an ultrasonic treatment and or a shaking treatment).

(Detection Apparatus)

From the viewpoint that a test substance can be detected with high sensitivity, the detector according to the disclosure preferably includes a specimen introduction unit into which a sample containing a test substance and water is introduced, a supply unit supplying a detection agent composition described below to the sample, a reaction unit in which the sample and the detection agent composition react with each other, and a detection unit detecting the reaction, and is more preferably a detection apparatus (online measuring instrument) that can be used online. Details of the detection agent composition used in the detection apparatus will be described below.

For example, in a case in which the test substance is an endotoxin and the response site includes a structure that emits fluorescence, as the detection apparatus, for example, a detection apparatus 100 illustrated in Fig. 2 can be suitably used.

The sample containing a test substance and water to be introduced into the specimen introduction unit can be replaced with a specimen of a test subject.

Fig. 2 is a schematic view illustrating an example of an endotoxin detection apparatus 100 of the disclosure. The endotoxin detection apparatus 100 of the disclosure includes a specimen introduction unit 200 into which a specimen 960 of a test subject is introduced, a supply unit 300 that supplies the above-described fluorescent substance 10 to the specimen, a reaction unit 400 in which the specimen 960 and the fluorescent substance 10 react with each other, and a detection unit 500 that detects fluorescence of the fluorescent substance 10 that has undergone the reaction. The

endotoxin detection apparatus 100 of the present example is configured by using a Flow Injection Analysis (FIA).

The specimen introduction unit 200 includes a carrier storage tank 210 that stores a carrier as a liquid conveying the specimen 960, a carrier feeding pump 220 that feeds the carrier, a carrier feeding path 230 through which the carrier flows, and a specimen introduction unit 240 into which the specimen 960 is introduced into the carrier feeding path 230. The carrier is not particularly limited as long as it is a liquid capable of conveying the specimen 960, and for example, pure water or various buffer solutions can be used according to the properties of the specimen 960 and the fluorescent substance 10, if appropriate. The specimen 960 is a liquid of a test subject whose presence or absence of an endotoxin 50 is verified, and examples of the specimen 960 include a liquid product such as purified water or injection water serving as a solvent such as an injection, an infusion solution, or a dialysis fluid, a biological fluid such as blood, saliva, or urine, or a diluent thereof. It is desirable that the specimen 960 collected from the specimen collection unit 700 provided in a production line (particularly, a purified water production facility and an injection water production facility) of the liquid product described above is directly and continuously introduced into the specimen introduction unit 240, but the collected specimen 960 may be introduced from the specimen introduction unit 240 each time of measurement.

In a case in which the endotoxin detection apparatus illustrated in Fig. 2 is directly connected to a purified water production facility or an injection water production facility described below and the endotoxin 50 is continuously detected, since an endotoxin at a lower concentration can be measured in a short time by using the detection method according to the disclosure, it is possible to ensure that the endotoxin concentration of purified water or injection water produced by the production method described below is less than a specified value. Fig. 3 illustrates a purified water production facility and an injection water production facility in the present invention.

The supply unit 300 includes a reagent storage tank 310 that stores a reagent solution containing the fluorescent substance 10, a reagent feeding pump 320 that feeds the reagent, a reagent feeding path 330 through which the reagent flows, and a mixing unit 340 that joins the carrier feeding path 230. In the mixing unit 340, the fluorescent substance 10 is supplied as a reagent solution to the specimen 960 and mixed. As described above, the fluorescent substance 10 has a structure in which the fluorescent site 20 and the recognition site 40, which recognizes a specific site 51 of the molecular structure are connected by the spacer 30. The meanings of the fluorescent site 20, the recognition site 40, and the spacer 30 are as described above.

In the reaction unit 400, in a case in which the endotoxin 50 is contained in the specimen 960, a reaction between the fluorescent substance 10 in the reagent and the endotoxin 50 occurs, and light is emitted from the fluorescent substance 10. As the reaction unit 400, a reaction coil adopted in a general detection system is used, but a simple capillary tube may be used as the reaction unit 400.

In the detection unit 500, the emission of the fluorescent substance 10 subjected to the above-described reaction is optically detected. As the detection unit 500, a general light emission detector can be used.

The endotoxin detection apparatus 100 of the disclosure is not limited to the configuration using the above-described FIA, and can also be realized by, for example, a configuration using a liquid flow analysis method (FCA) including a continuous flow analysis method (CFA), a sequential injection analysis method (SIA), and a reverse flow injection analysis method (r-FIA).

In the endotoxin detection apparatus 100 of the disclosure, it is desirable to use, as the fluorescent substance 10, dpa-HCC that reacts with a phosphoric acid group as the specific site 51 or C4-APB that reacts with a sugar chain moiety as the specific site 51.

(Purified Water Production Facility)

A purified water production facility according to the disclosure preferably includes: a purified water production unit using at least one of a reverse osmosis membrane device, which performs reverse osmosis filtration of raw water, or an ion exchange device, which performs ion exchange of raw water, to obtain purified water from the raw water; a specimen collection unit collecting a specimen of the test subject from the purified water; and the above-described detection apparatus.

Fig. 3 is a schematic view of a schematic configuration of an example of a purified water production facility 600 according to the disclosure. The purified water production facility 600 of the disclosure includes a purified water production unit 610 that obtains purified water 920 from raw water 900, a specimen collection unit 700 that collects a specimen 960 of a test subject from the purified water 920, and the above-described endotoxin detection apparatus 100.

The purified water production unit 610 is, for example, a facility that removes impurities from the raw water 900 by at least one of a reverse osmosis membrane device (610), which performs reverse osmosis filtration of the raw water 900 or an ion exchange device (620), which performs ion exchange of raw water, to produce the purified water 920. As the ion exchange device, for example, an electrodeionization device or a mixed bed ion exchange resin device can be used. The purified water production unit 610 may further include an ultrafiltration membrane device 630 or an ultraviolet irradiation device. The produced purified water 920 may be supplied to an injection water production facility 800 or may be supplied to a use place (not illustrated).

The specimen collection unit 700 is provided downstream of the purified water production unit 610 and in the middle of the conveyance line of the purified water 920. The purified water 920 collected by the specimen collection unit 700 is introduced, as the specimen 960 of the test subject, into the endotoxin detection apparatus 100 from the above-described specimen introduction unit 240 (see Fig. 2). Details of the endotoxin detection apparatus 100 will be described below. As a result, the specimen 960 of the test subject collected from the purified water 920 is subjected to the above-described endotoxin detection method.

With the above configuration, in the purified water production facility 600 of the disclosure, the specimen 960 is collected from the produced purified water 920 as needed, and the endotoxin detection apparatus 100 can detect the endotoxin 50 in real time. In a case in which the concentration of the endotoxin 50 exceeds a predetermined standard value, for example, the purified water production facility 600 is immediately stopped, and appropriate measures such as part replacement or repair can be taken.

(Injection Water Production Facility)

An injection water production facility according to the disclosure preferably includes: a purified water production unit using at least one of a reverse osmosis membrane device, which performs reverse osmosis filtration of raw water, or an ion exchange device, which performs ion exchange of raw water, to obtain purified water from the raw water; an injection water production unit using a polymer membrane filtration device filtering the purified water or a distiller distilling the purified water to obtain injection water from the purified water; an injection water tank maintaining the injection water in a heated state and storing the injection water; a delivery line delivering the injection water stored in the injection water tank to a predetermined use place; a specimen collection unit collecting a specimen of the test subject from the injection water; and the above-described detection apparatus.

Fig. 3 is a schematic view of a schematic configuration of an example of an injection water production facility 800 according to the disclosure. The injection water production facility 800 according to the disclosure includes a purified water production unit 610 that obtains purified water 920 from raw water 900, an injection water production unit 810 that obtains injection water 940 from the purified water 920, an injection water tank 820 that maintains the injection water 940 in a heated state and stores the injection water 940, a delivery line 830 that delivers the injection water 940 stored in the injection water tank 820 to a predetermined use place 850, a specimen collection unit 700 that collects a specimen 960 of a test subject from the injection water 940, and the above-described endotoxin detection apparatus 100.

In the injection water production unit 810, the purified water 920 is filtered by a polymer membrane filtration device (preferably an ultrafiltration membrane device) 870 to highly remove impurities such as the endotoxin 50, thereby producing the injection water 940. A distiller may be used instead of the polymer membrane filtration device.

In a case in which the purified water production unit 600 is provided with the endotoxin detection apparatus 100 and the endotoxin concentration of the purified water 920 is known, the operating conditions of the injection water production unit 810 may be adjusted accordingly. For example, in a case in which it is known that the endotoxin concentration of the purified water 920 is sufficiently low, the endotoxin removal capability may be lowered by that amount to increase the amount of permeated water of the polymer membrane filtration device, and the production amount of the injection water 940 may be increased.

In the injection water tank 820, the produced injection water is heated by a heat exchanger 880 to prevent growth of bacteria and the like in the injection water 940, and a temperature state of, for example, 60°C or higher, desirably 70°C or higher is maintained in order to maintain a clean state. In order to prevent retention of the injection water 940 while maintaining this temperature state, it is desirable to form the injection water tank 820 using a circulation pipe. The circulation pipe may be provided with a heat exchanger 890. A heating device such as a heater may be installed in the injection water tank 820.

The delivery line 830 delivers the injection water 940 stored in the injection water tank 820 to the predetermined use place 850 as needed or as if necessary. As the predetermined use place 850, for example, various facilities according to the use of the injection water 940, such as a packaging facility of the injection water 940 or a production facility of an injection solution, are assumed.

The specimen collection unit 701 is provided at a predetermined place on the downstream side of the injection water production unit 810 in the injection water production facility 800. The injection water 920 collected by the specimen collection unit 701 is introduced, as the specimen 960 of the test subject, into the endotoxin detection apparatus 100 from the above-described specimen introduction unit 240 (see Fig. 2). Details of the endotoxin detection apparatus 100 will be described below. As a place where the specimen collection unit 701 is provided, the specimen collection unit 701 may be provided either on the downstream side of the injection water production unit 810 and on the upstream side of the injection water tank 820, directly in the injection water tank 820 (for example, in the middle of the circulation pipe) or in the middle of the delivery line 830. In any case, the specimen 960 of the test subject collected from the injection water 940 is subjected to the above-described endotoxin detection method.

In a case in which the specimen collection unit 701 is provided on the downstream side of the injection water

production unit 810 and on the upstream side of the injection water tank 820, it is possible to substantially constantly confirm whether or not the endotoxin concentration of the injection water 940 immediately after being produced by the injection water production unit 810 satisfies, for example, the standard (0.25 EU/mL) described in the Japanese Pharmacopoeia. If this standard is not satisfied by any chance, it is possible to prevent contamination of the injection water 940 in a state of satisfying the standard stored in the injection water tank 820 on the downstream side at that time by immediately stopping the supply of the injection water 940 to the downstream side. The phrase "substantially constantly" refers to, for example, measurement in which the repeated measurement time is within 30 minutes, preferably within 10 minutes. In consideration of the amount of water held by the device on the downstream side and the amount of water produced by the device, by repeating the measurement at these frequencies, operation can be conducted without stopping the production of injection water due to contamination caused by water quality deterioration due to trouble.

In a case in which the specimen collection unit 701 is directly provided in the injection water tank 820, it is possible to substantially constantly monitor whether or not the circulating and stored injection water 940 satisfies the above standard. For example, only in a case in which it is confirmed that the above standard is satisfied, the injection water 940 may be supplied to the delivery line 830, and in a case in which the above standard is not satisfied, the injection water 940 may be returned to the upstream side of the injection water production unit 810 using a return line (not illustrated) and reprocessed in the injection water production unit 810.

Since the endotoxin detection apparatus 100, which is an example of the detection apparatus according to the disclosure, has sufficient measurement accuracy, it is possible to confirm not only a trouble of the device at the preceding stage but also a sign of the trouble by substantially constantly monitoring. For example, in a case in which the endotoxin measured value of the injection water 940 to be produced satisfies the standard value of the endotoxin but shows a slight rising tendency, this is often a sign of deterioration of the performance of the device at the preceding stage, and if this is left as it is, the injection water 940 to be produced does not satisfy the standard value of the endotoxin in some cases. That is, the trouble can be avoided by detecting the trouble of an injection water production apparatus in advance. For example, it is possible to cope with such a trouble by changing the operating conditions of the device at the preceding stage, performing maintenance, and the like.

In a case in which the specimen collection unit 701 is provided in the delivery line 830, it is possible to directly monitor the endotoxin concentration of the injection water 940 at the time when the injection water 940 is actually used. For example, it is also conceivable to use a batch type in which a tank is installed immediately before the use place 850, injection water is stored in the tank, the injection water is supplied to the use place 850, and the injection water is stored again in a case in which the tank becomes empty. By checking the endotoxin concentration of the injection water in the tank with the endotoxin detection apparatus 100 for each batch, it is possible to guarantee the use of the injection water whose endotoxin concentration satisfies the control standard.

All of the three places described above have an advantage of installing the specimen collection unit 700 and are preferable installation places; however, it is most preferable to provide the specimen collection unit 700 on the downstream side of the injection water production unit 810 and on the upstream side of the injection water tank 820 in that contamination of the injection water 940 with the endotoxin 50 can be detected at the earliest. If possible, the specimen collection unit 700 may be installed at two or more of the three places.

With the above configuration, in the injection water production facility 800 of the disclosure, the specimen 960 is collected from the produced injection water 940 as needed, and the endotoxin detection apparatus 100 can detect the endotoxin 50 in real time. In a case in which the concentration of the endotoxin 50 exceeds a predetermined standard value, the injection water production facility 800 is immediately stopped, and appropriate measures such as part replacement or repair can be taken. As a result, the injection water 940 having the required water quality can be stably produced.

In the injection water production facility 800 of the disclosure, since the impurity concentration of the injection water 940 to be the specimen 960 is sufficiently small and the influence of other impurities can be minimized, the endotoxin concentration can be measured particularly accurately. Since the viscosity of the injection water 940 to be the specimen 960 is sufficiently low, it is not particularly necessary to dilute the injection water 940 with a carrier solution or the like, and the injection water 940 can be directly fed to the endotoxin detection apparatus 100. As a result, there is also an advantage that the carrier solution is not required and the specimen 960 is not unnecessarily diluted with the carrier.

The specimen 960 collected by the specimen collection unit 701 is collected in a heated state. The specimen 960 having such a high temperature is preferably cooled to room temperature by a heat exchanger or the like before being supplied to the endotoxin detection apparatus 100.

(Purified Water Production Method)

A purified water production method according to the disclosure preferably includes a step of treating raw water by at least one of reverse osmosis filtration or ion exchange to obtain purified water, and a measurement step of measuring

a specimen of a test subject collected from the purified water using the test substance detection method according to the disclosure.

(Injection Water Production Method)

An injection water production method according to the disclosure preferably includes
a step of treating raw water by at least one of reverse osmosis filtration or ion exchange to obtain purified water, a step of obtaining injection water from the purified water by filtration of the purified water using polymer membrane filtration or by distillation of the purified water, and a measurement step of measuring a specimen of the test subject collected from the injection water using the test substance detection method according to the disclosure.
In the purified water production method and the injection water production method according to the disclosure, a preferred aspect of the detection method used in the measurement step is the same as the preferred aspect of the detection method according to the disclosure.
In the purified water production method and the injection water production method according to the disclosure, from the viewpoint that a test substance can be detected with high sensitivity, it is still more preferable to further include, as the measurement step, a measurement step of measuring a specimen of a test subject collected from the purified water using the measurement device.

(Detection Agent Composition)

A detection agent composition according to the disclosure contains a compound represented by the following Formula (1) and a surfactant.

A-L-Y Formula (1)

Formula (1)

In Formula (1), A represents a response site which is a group containing a conjugated multiple bond, L represents a single bond or a spacer having a chain length of from 1 to 10 atoms, and Y represents a recognition site which is an organic group.
The compound represented by Formula (1) contained in the detection agent composition according to the disclosure has the same meaning as the compound represented by Formula (1) used in the detection method according to the disclosure, and a preferred aspect is also the same.
In the detection agent composition according to the disclosure, the content of the compound represented by Formula (1) is preferably from $1\times10^{-5}$% by mass to $1\times10^{-1}$% by mass and more preferably from $1\times10^{-4}$% by mass to $1\times10^{-2}$% by mass with respect to the total mass of the composition.
The detection agent composition according to the disclosure may contain one kind of the compound represented by Formula (1) singly, or may contain two or more kinds thereof.
The surfactant contained in the detection agent composition according to the disclosure has the same meaning as the surfactant used in the detection method according to the disclosure, and a preferred aspect is also the same.
In the detection agent composition according to the disclosure, the content of the surfactant is preferably from 0.1 times to 1,000 times, more preferably from 0.5 times to 1,000 times, and still more preferably from 1 time to 100 times the critical micelle concentration. Alternatively, the content is preferably from $1\times10^{-5}$% by mass to 40% by mass and more preferably from $1\times10^{-3}$% by mass to 2% by mass with respect to the total mass of the composition.
The detection agent composition according to the disclosure may further contain components (other components) other than the compound represented by the above Formula (1) and the surfactant.
Examples of the other components include a stabilizer, a buffer solution, and an ionic strength adjusting agent.
The detection agent composition according to the disclosure can be suitably used for the detection apparatus described above, and can also be suitably used for a detection apparatus used in a purified water production facility or an injection water production facility from the viewpoint that a test substance can be detected with high sensitivity. From the viewpoint that a test substance can be detected with high sensitivity, the detection agent composition according to the disclosure is preferably used for detecting an alkali metal, a transition metal, a surfactant, a bacterium, a phospholipid, or a glycolipid, and more preferably used for detecting an endotoxin.

Examples

Hereinafter, the disclosure will be described more specifically with reference to Examples. The disclosure is

not limited to these Examples at all.

(Example 1) Measurement of Endotoxin by $Cu^{2+}$-dpa-C4Py/DDAB

Measurement of an endotoxin was attempted using dpa-HCC. An experimental method was as follows.
As a measurement sample, the following components were mixed so that the endotoxin concentration was from 0.0 EU/mL to 100 EU/mL, and the mixture was prepared in a 10 mL volumetric flask to obtain a measurement sample.

<Composition of Measurement Sample>

· Compound represented by Formula (1) (specific compound) dpa-C4Py: 0.01 mM (mmol/L, the same applies hereinafter)
· Surfactant

DDAB: 0.1 mM (dodecyldimethylammonium bromide, cationic surfactant, HLB: 18.1) (subjected to ultrasonic treatment (condition: frequency 20 kHz, treatment time: 3 min, apparatus: Model No. 250, manufactured by BRANSON Corporation) to form vesicles) Note that, when the measurement sample was observed with a TEM (magnification: 40,000 times, acceleration voltage: 75.0 kV, apparatus: Model No. H-7100, manufactured by Hitachi, Ltd.), it was confirmed that vesicles were formed.

· Metal compound (metal ion)
$Cu(NO_3)_2$: 0.01 mM
· HEPES buffer solution (pH 7.4): 5.0 mM
· Test substance
Endotoxin standard solution (Model No.: 293-16541 (derived from E. Coli UKTB strain), manufactured by FUJIFILM Wako Pure Chemical Corporation): 0.0 EU/mL, 10.0 EU/mL, 100.0 EU/mL

Each sample prepared above in which the endotoxin concentration was changed to 0 EU/mL, 10 EU/mL, and 100 EU/mL was taken in a quartz cell having an optical path length of 1 cm, and a fluorescence spectrum was measured with a fluorescence spectrophotometer (Model No.: F-7000, manufactured by Hitachi High-Tech Corporation). The measurement conditions were as follows.

Excitation wavelength: 350 nm
Start wavelength: 360 nm
End wavelength: 640 nm

According to the present measurement conditions, measurement of all samples could be completed within 5 minutes.
The fluorescence spectrum of each sample in which the endotoxin concentration was changed to 0 EU/mL, 10 EU/mL, and 100 EU/mL is as shown in Fig. 4. In Fig. 4, the vertical axis represents fluorescence intensity, and the horizontal axis represents wavelength (nm).
Each sample in which the endotoxin concentration was changed to from 0 EU/mL to 100 EU/mL showed a peak of fluorescence intensity at a wavelength of 377 nm. It is found that the peak intensity of the fluorescence intensity is high at a wavelength of 377 nm in proportion to the endotoxin concentration.
A calibration curve of a fluorescence intensity at a wavelength of 377 nm with respect to each endotoxin concentration was prepared (Fig. 5). As shown in Fig. 5, it is found that the endotoxin can be detected at a wavelength of 377 nm even in a case in which the endotoxin concentration is from 0.1 EU/mL to 20 EU/mL.

<<Relationship between dpa-C4Py and Concentration of DDAB>>

A sample containing a surfactant: DDAB at a concentration of from 0 M to $10\times10^{-5}$ M, dpa-C4Py, and an endotoxin (100 EU/mL) was prepared, and the fluorescence intensity at room temperature (25°C, the same applies hereinafter) was measured. As a comparative control, a sample containing no endotoxin (sample containing a surfactant: DDAB at a concentration of from 0 M to $10\times10^{-5}$ M and dpa-C4Py) was prepared, and the fluorescence intensity at room temperature was measured. The results are shown in Fig. 6.
From Fig. 6, when the case of a DDAB concentration of $2\times10^{-5}$ M to $4\times10^{-5}$ M and the case of a DDAB

concentration of 0 are compared with each other, the fluorescence intensity was from 3 to 35 times in a case in which an endotoxin was present. When the case of a DDAB concentration of $4\times10^{-5}$ M to $10\times10^{-5}$ M and the case of a DDAB concentration of 0 are compared with each other, the fluorescence intensity was from 35 to 90 times in a case in which an endotoxin was present. When the case of a DDAB concentration of $10\times10^{-5}$ M or more (not shown in a case in which the DDAB concentration was more than $10\times10^{-5}$ M) and the case of a DDAB concentration of 0 are compared with each other, the fluorescence intensity was from 90 to 300 times in a case in which an endotoxin was present.

It is found that the fluorescence intensity remarkably increased in the sample containing an endotoxin in a case in which DDAB was equal to or more than the critical micelle concentration ($4\times10^{-5}$ M).

The ratio of the fluorescence intensity at a DDAB concentration of $10\times10^{-5}$ M to the fluorescence intensity at a DDAB concentration of 0 is shown in Table 1 described below.

(Example 2) Measurement of Endotoxin by $Zn^{2+}$-dpa-C4Py/DDAB

A measurement sample was prepared in the same manner as in Example 1, except that in Example 1, $Cu(NO_3)_2$ was changed to $Zn(NO_3)_2$ and the endotoxin concentration was changed to 100 EU/mL, and the fluorescence intensity was measured under the same measurement conditions. The results are shown in Fig. 7.

In Fig. 7, the vertical axis represents fluorescence intensity, and the horizontal axis represents wavelength (nm). In Fig. 7, it is found that the fluorescence intensity at a wavelength of 377 nm increases in the sample containing an endotoxin.

(Example 3) Measurement of Endotoxin by $Cu^{2+}$-dpa-C4Py/CTAB

A measurement sample in which the endotoxin concentration was changed to from 0 EU/mL to 100 EU/mL was prepared in the same manner as in Example 1, except that in Example 1, the surfactant was changed to CTAB (cetyltrimethylammonium bromide: HLB 10, cationic surfactant): $2.0\times10^{-2}$ M. The fluorescence spectrum of each sample in which the endotoxin concentration was changed to 0 EU/mL, 10 EU/mL, and 100 EU/mL is as shown in Fig. 8.

A calibration curve of a fluorescence intensity at a wavelength of 377 nm with respect to each endotoxin concentration was prepared (Fig. 9). As shown in Fig. 9, it is found that the endotoxin can be detected even in a case in which the endotoxin concentration is from 0.1 EU/mL to 20 EU/mL.

«Relationship between dpa-C4Py and Concentration of CTAB>>

A sample containing a surfactant: CTAB at a concentration of from 0 mM to 2 mM, dpa-C4Py, and an endotoxin (100 EU/mL) was prepared, and the fluorescence intensity at room temperature was measured. As a comparative control, a sample containing no endotoxin (sample containing each surfactant: CTAB at a concentration of from 0 mM to 2 mM and dpa-C4Py) was prepared, and the fluorescence intensity at room temperature was measured. The results are shown in Fig. 10. In Fig. 10, the vertical axis represents fluorescence intensity, and the horizontal axis represents the concentration (mM) of the surfactant.

It is found that the fluorescence intensity remarkably increased in the sample containing an endotoxin in a case in which CTAB was equal to or more than the critical micelle concentration (1 mM).

From Fig. 10, the results of comparing the fluorescence intensity in the case of a surfactant concentration of $2.0\times10^{-2}$ M with the fluorescence intensity in the case of a surfactant concentration of 0 mM are shown in Table 1.

(Example 4) Measurement of Endotoxin by $Zn^{2+}$-dpa-C4Py/DOPC

A measurement sample was prepared in the same manner as in Example 1, except that in Example 1, $Cu(NO_3)_2$ was changed to $Zn(NO_3)_2$, and the type of surfactant was changed to DOPC (1,2-dilinoleoyl-sn-glycero-3-phosphocholine, HLB: 7.2, amphoteric surfactant), and the fluorescence intensity was measured under the same conditions.

The results of comparing the fluorescence intensity in the case of a surfactant concentration of $10\times10^{-5}$ M with the fluorescence intensity in the case of a surfactant concentration of 0 mM are shown in Table 1.

(Example 5) Measurement of Lauryl Phosphate by $Cu^{2+}$-dpa-azo/PS

A measurement sample was prepared in the same manner as in Example 1, except that in Example 1, the specific compound was changed to dpa-azo, the type of surfactant was changed to PS (polysorbate, manufactured by KANTO CHEMICAL CO., INC., polyoxyethylene sorbitan monooleate, HLB: 15, non-ionic surfactant), and the test substance was changed to lauryl phosphate (concentration: 0.01 mM), and the fluorescence intensity was measured under the same conditions.

The results of comparing the fluorescence intensity in the case of a surfactant concentration of $2.0\times10^{-2}$ M with the fluorescence intensity in the case of a surfactant concentration of 0 mM are shown in Table 1.

(Example 6) Measurement of Phosphatidylcholine by $Cu^{2+}$-dpa-C6Py/DOPC

A measurement sample was prepared in the same manner as in Example 1, except that in Example 1, the specific compound was changed to dpa-C6Py, the type of surfactant was changed to DOPC (1,2-dilinoleoyl-sn-glycero-3-phosphocholine, HLB: 7.2, amphoteric surfactant), and the test substance was changed to phosphatidylcholine (concentration: 0.01 mM), and the fluorescence intensity was measured under the same conditions.
The results of comparing the fluorescence intensity in the case of a surfactant concentration of $10\times10^{-5}$ M with the fluorescence intensity in the case of a surfactant concentration of 0 mM are shown in Table 1.

(Example 7) Measurement of Galactolipid by $Cu^{2+}$-C4-APB/CTAB

A measurement sample was prepared in the same manner as in Example 1, except that in Example 1, the specific compound was changed to dpa-APB, the type of surfactant was changed to CTAB (cetyltrimethylammonium bromide, HLB: 10, cationic surfactant), and the test substance was changed to galactolipid (concentration: 0.01 mM), and the fluorescence intensity was measured under the same conditions.
The results of comparing the fluorescence intensity in the case of a surfactant concentration of $2.0\times10^{-2}$ M with the fluorescence intensity in the case of a surfactant concentration of 0 mM are shown in Table 1.

(Example 8) Measurement of E. Coli by $Cu^{2+}$-dpa-C6Py/CTAB

A measurement sample was prepared in the same manner as in Example 1, except that in Example 1, the specific compound was changed to dpa-C6Py, the type of surfactant was changed to CTAB (cetyltrimethylammonium bromide, HLB: 10, cationic surfactant), and the test substance was changed to E. coli (strain name: K12W3110, concentration: 100 CFU/mL), and the fluorescence intensity was measured under the same conditions.
The results of comparing the fluorescence intensity in the case of a surfactant concentration of $2.0\times10^{-2}$ M with the fluorescence intensity in the case of a surfactant concentration of 0 mM are shown in Table 1.

(Comparative Example 1) Measurement of Endotoxin by $Cu^{2+}$-dpa-C4Py

A measurement sample was prepared in the same manner as in Example 1, except that in Example 1, the surfactant was not contained and the endotoxin concentration was set to 100 EU/mL. As a comparative control, a comparative control sample not containing an endotoxin in the above composition and a comparative control sample not containing an endotoxin and a metal complex in the above composition were prepared, respectively.
The fluorescence intensity of each of the measurement sample and the comparative control sample was measured in the same manner as in Example 1.
In the measurement of Comparative Example 1 containing no surfactant, a change in fluorescence intensity could be hardly confirmed even in a case in which an endotoxin was contained.

(Comparative Example 2) Measurement of Endotoxin by $Zn^{2+}$-dpa-C4Py

A measurement sample and a comparative control sample were prepared in the same manner as in Comparative Example 1, except that in Comparative Example 1, $Cu(NO_3)_2$ was changed to $Zn(NO_3)_2$, and the fluorescence intensity of each of the measurement sample and the comparative control sample was measured by the same method as in Example 1. In the measurement of Comparative Example 1 containing no surfactant, a change in fluorescence intensity could not be confirmed even in a case in which an endotoxin was contained.

<Quantitability of Endotoxin by FIA>

The quantitativity of endotoxin by an endotoxin detection apparatus using a flow injection analysis method (FIA) as illustrated in Fig. 2 was verified. A specific verification method is as follows.
Ultrapure water was used as a carrier. As the fluorescent substance, dpa-C4Py was used, and the reagent was prepared with the following composition, and then the pH was adjusted to 7.4.

dpa-C4Py: 0.02 mM
DDAB: 0.2 mM

NaCl: 20 mM
HEPES: 10 mM
$Cu(NO_3)_2$: 0.02 mM

As a specimen containing an endotoxin as a test subject, aqueous solutions obtained by diluting a standard endotoxin to 0.2 EU/mL, 2 EU/mL, 20 EU/mL, and 200 EU/mL were prepared. This specimen was added to the carrier by a syringe from the specimen introduction unit.

The measurement conditions were as follows.

Carrier flow rate: 0.5 mL/min
Reagent flow rate: 0.5 mL/min
Excitation wavelength: 350 nm
Fluorescence wavelength: 377 nm

The fluorescence intensity peaked at 40 seconds after addition of the specimens at any of endotoxin concentrations. From this result, it was inferred that the endotoxin concentration over time can be measured by the endotoxin detection apparatus using FIA. It has become possible to quickly measure a specimen sample online within 3 minutes. That is, it was confirmed that the specimen having an endotoxin concentration of from 0.2 EU/mL to 200 EU/mL used in this Example can be measured by the measurement conditions selected in this Example.

It was confirmed that, in a case in which the detection apparatus is used in the pure water production facility and the injection water production facility, it was possible to quantify 0.01 EU/mL to 10 EU/mL, which is an endotoxin concentration sufficient for prophylactically controlling a value of less than 0.25 EU/mL, which is the control standard of the endotoxin in the injection water. Also in the purified water production method and the injection water production method according to the disclosure, it was confirmed that the endotoxin concentration of from 0.01 EU/mL to 10 EU/mL can be quantified in the same manner.

From the above results, the increase ratios of the optical intensity depending on the presence or absence of addition of the surfactant are summarized as in Table 1. It can be said that the test substance can be detected with high sensitivity as the increase ratio of the optical intensity is larger.

[Table 1]

| | Test substance | | Specific compound (probe) | | Surfactant | | | Detection | | Optical intensity increase ratio |
|---|---|---|---|---|---|---|---|---|---|---|
| | Name | Concentration mM | Type | Concentration | Name | Concentration | Type of molecular assembly | Method | Wavelength | |
| Example 1 | Endotoxin | 100 EU/ml | $Cu^{2+}$-dpa-C4Py | 0.01 mM | DDAB | $10\times10^{-5}$ M | Vesicle | Fluorescence | 377 nm | 35 times |
| Example 2 | Endotoxin | 100 EU/ml | $Zn^{2+}$-dpa-C4Py | 0.01 mM | DDAB | $10\times10^{-5}$ M | Vesicle | Fluorescence | 377 nm | 12 times |
| Example 3 | Endotoxin | 100 EU/ml | $Cu^{2+}$-dpa-C4Py | 0.01 mM | CTAB | $2\times10^{-2}$ M | Micelle | Fluorescence | 377 nm | 170 times |
| Example 4 | Endotoxin | 100 EU/ml | $Zn^{2+}$-dpa-C4Py | 0.01 mM | DOPC | $10\times10^{-5}$ M | Vesicle | Fluorescence | 377 nm | 70 times |
| Example 5 | Lauryl phosphate | 0.01 mM | $Cu^{2+}$-dpa-azo | 0.01 mM | PS | $2\times10^{-2}$ M | Micelle | Fluorescence | 490 nm | 13 times |
| Example 6 | Phosphatidylcholine | 0.01 mM | $Cu^{2+}$-dpa-C6Py | 0.01 mM | DOPC | $10\times10^{-5}$ M | Vesicle | Fluorescence | 377 nm | 10 times |
| Example 7 | Galactolipid | 0.01 mM | C4-APB | 0.01 mM | CTAB | $2\times10^{-2}$ M | Micelle | Fluorescence | 377 nm | 150 times |
| Example 8 | E. coli | 100 CFU/mL | $Cu^{2+}$-dpa-C6Py | 0.01 mM | CTAB | $2\times10^{-2}$ M | Micelle | Fluorescence | 377 nm | 22 times |

From the above results, it is found that in Examples 1 to 8, which are the detection method according to the disclosure and the detection agent composition according to the disclosure, the test substance can be detected with approximately from 10 times to 170 times higher sensitivity as compared with Comparative Examples 1 and 2.

DESCRIPTION OF REFERENCE NUMERALS

| | |
|---|---|
| 10 | Fluorescent substance |
| 20 | Fluorescent site |
| 30 | L (single bond or spacer) |
| 40 | Recognition site |
| 50 | Endotoxin |
| 51 | Specific site |
| 100 | Endotoxin detection apparatus |
| 200 | Specimen introduction unit |
| 210 | Carrier storage tank |
| 220 | Carrier feeding pump |
| 230 | Carrier feeding path |
| 240 | Specimen introduction unit |
| 300 | Supply unit |
| 310 | Reagent storage tank |
| 320 | Reagent feeding pump |
| 330 | Reagent feeding path |
| 340 | Mixing unit |
| 400 | Reaction unit |
| 500 | Detection unit |
| 600 | Purified water production facility |
| 610 | Reverse osmosis membrane device (RO) |
| 620 | Electrodeionization device (EDI) |
| 630 | Ultrafiltration device (UF) |
| 700 | Specimen collection unit |
| 701 | Specimen collection unit |
| 780 | Use place (injection water) |
| 800 | Injection water production facility |
| 810 | Injection water (WFI) production apparatus |
| 820 | Injection water tank |
| 830 | Delivery line |
| 850 | Use place |
| 860 | Tank |
| 870 | Ultrafiltration device (UF) |
| 880, 890 | Heat exchanger |
| 900 | Raw water |
| 920 | Purified water |
| 940 | Injection water |
| 960 | Specimen |

## Claims

**1.** A test substance detection method comprising:

a step A of mixing a sample containing a test substance and water, a compound having a structure including a response site and a recognition site, and a surfactant; and
a step B of detecting a response at the response site arising from interaction between the recognition site and the test substance.

**2.** The test substance detection method according to claim 1, wherein the compound has a structure in which the

response site and the recognition site are connected via a spacer.

3. The test substance detection method according to claim 1 or 2, wherein the compound is a compound represented by the following Formula (1):

A-L-Y Formula (1)

wherein A represents a response site which is a group containing a conjugated multiple bond, L represents a single bond or a spacer having a chain length of from 1 to 10 atoms, and Y represents a recognition site which is a group interacting with a test substance.

4. The test substance detection method according to claim 3, wherein the L is a linear saturated alkyl chain.

5. The test substance detection method according to claim 3 or 4, wherein Y in Formula (1) is a metal coordinating group or a group containing an acid group.

6. The test substance detection method according to claim 5, wherein the metal coordinating group or the group containing an acid group is a group represented by the following Formula (Y1) or Formula (Y2):

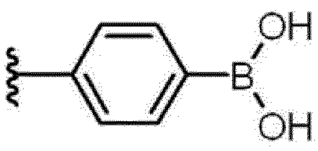

Formula (Y1)

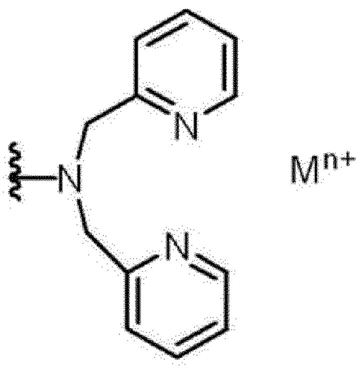

Formula (Y2)

wherein a wavy line represents a linking site with L in Formula (1), $M^{n+}$ represents a metal ion, and n represents a natural number.

7. The test substance detection method according to claim 6, wherein the metal ion is a copper ion ($Cu^{2+}$), a nickel ion ($Ni^{2+}$), or a cobalt ion ($Co^{2+}$).

8. The test substance detection method according to any one of claims 1 to 7, wherein an HLB value of the surfactant is from 6 to 40.

9. The test substance detection method according to any one of claims 1 to 8, wherein the surfactant is a cationic surfactant, an amphoteric surfactant, or a non-ionic surfactant.

10. The test substance detection method according to any one of claims 1 to 9, wherein the compound is a compound capable of forming a molecular assembly with the surfactant.

11. The test substance detection method according to any one of claims 1 to 10, wherein the test substance contains at least one of a phospholipid or a glycolipid.

12. The test substance detection method according to any one of claims 1 to 11, wherein the test substance is an endotoxin, and

the compound is a compound represented by the following Formula (1A) or C4-APB:

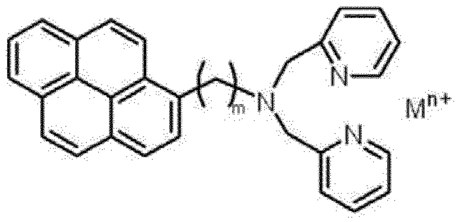

Formula (1A)

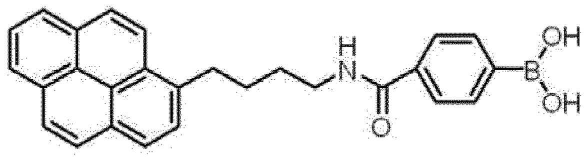

(C4-APB)

wherein m represents from 0 to 6, $M^{n+}$ represents a metal ion, and n represents a natural number.

**13.** A detection agent composition comprising:

a compound represented by the following Formula (1); and
a surfactant:

A-L-Y Formula (1)

wherein A represents a response site which is a group containing a conjugated multiple bond, L represents a single bond or a spacer having a chain length of from 1 to 10 atoms, and Y represents a recognition site which is an organic group.

**14.** The detection agent composition according to claim 13, wherein the L is a linear saturated alkyl chain.

**15.** The detection agent composition according to claim 13 or 14, wherein Y in Formula (1) is a metal coordinating group or a group containing an acid group.

**16.** The detection agent composition according to claim 15, wherein the metal coordinating group or the group containing an acid group is a group represented by the following Formula (Y1) or Formula (Y2):

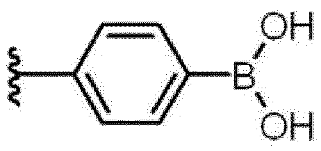

Formula (Y1)

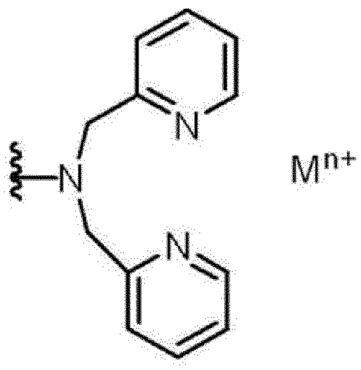

Formula (Y2)

wherein a wavy line represents a linking site with L in Formula (1), $M^{n+}$ represents a metal ion, and n represents a natural number.

**17.** The detection agent composition according to claim 16, wherein the metal ion is a copper ion ($Cu^{2+}$), a nickel ion ($Ni^{2+}$), or a cobalt ion ($Co^{2+}$).

**18.** The detection agent composition according to any one of claims 13 to 17, wherein an HLB value of the surfactant is from 6 to 40.

**19.** The detection agent composition according to any one of claims 13 to 18, wherein the surfactant is a cationic surfactant, an amphoteric surfactant, or a non-ionic surfactant.

**20.** A detection apparatus comprising:

a specimen introduction unit into which a sample containing a test substance and water is introduced;
a supply unit supplying the detection agent composition according to any one of claims 13 to 19 to the sample;
a reaction unit in which the sample and the detection agent composition react with each other; and
a detection unit detecting the reaction.

**21.** A purified water production facility comprising:

a purified water production unit using at least one of a reverse osmosis membrane device, which performs reverse osmosis filtration of raw water, or an ion exchange device, which performs ion exchange of raw water, to obtain purified water from the raw water;
a specimen collection unit collecting a specimen of the test subject from the purified water; and
the detection apparatus according to claim 20.

**22.** An injection water production facility comprising:

a purified water production unit using at least one of a reverse osmosis membrane device, which performs reverse osmosis filtration of raw water, or an ion exchange device, which performs ion exchange of raw water, to obtain purified water from the raw water;
an injection water production unit using a polymer membrane filtration device filtering the purified water or a distiller distilling the purified water to obtain injection water from the purified water;
an injection water tank maintaining the injection water in a heated state and storing the injection water;
a delivery line delivering the injection water stored in the injection water tank to a predetermined use place;
a specimen collection unit collecting a specimen of the test subject from the injection water; and
the detection apparatus according to claim 20.

**23.** The injection water production facility according to claim 22, wherein the specimen collection unit collects the specimen directly from the injection water tank or from the delivery line on a downstream side of the injection water production unit and on an upstream side of the injection water tank.

**24.** A purified water production method comprising:

a step of treating raw water by at least one of reverse osmosis filtration or ion exchange to obtain purified water; and
a measurement step of measuring a specimen of a test subject collected from the purified water using the detection method according to any one of claims 1 to 12.

**25.** The purified water production method according to claim 24, wherein the measurement step includes a measurement step of measuring a specimen of a test subject collected from the purified water using the detection apparatus according to claim 20.

**26.** An injection water production method comprising:

a step of treating raw water by at least one of reverse osmosis filtration or ion exchange to obtain purified water;
a step of obtaining injection water from the purified water by filtration of the purified water using polymer membrane filtration or by distillation of the purified water; and
a measurement step of measuring a specimen of the test subject collected from the injection water using the detection method according to any one of claims 1 to 12.

**27.** The injection water production method according to claim 25, wherein the measurement step includes a measurement step of measuring a specimen of a test subject collected from the purified water using the detection apparatus according to claim 20.

FIG.1

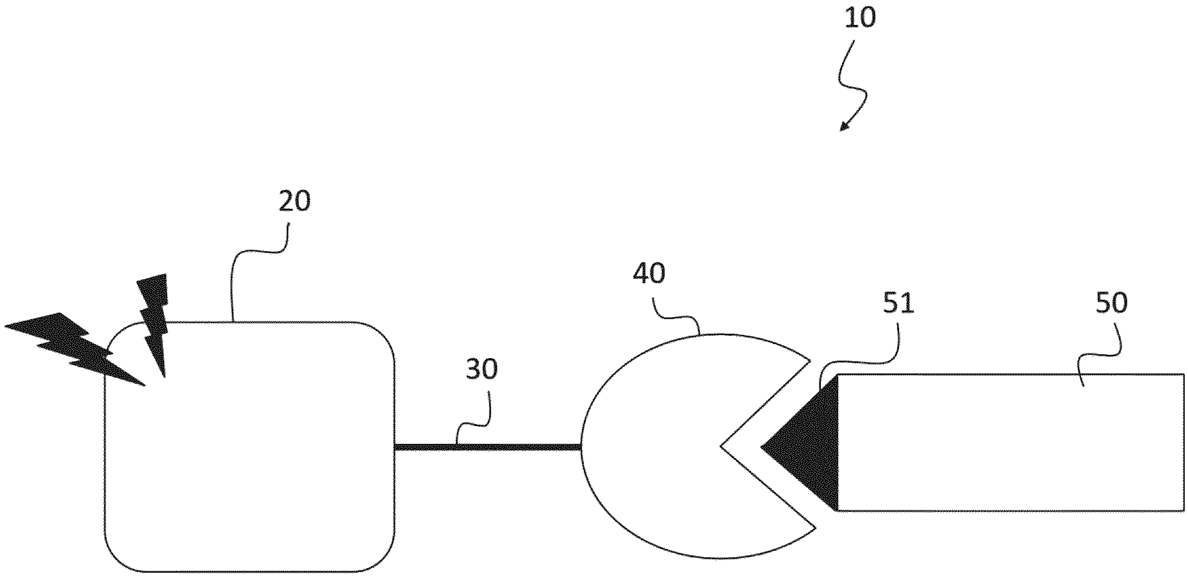
10
20
40
51
50
30

FIG.2

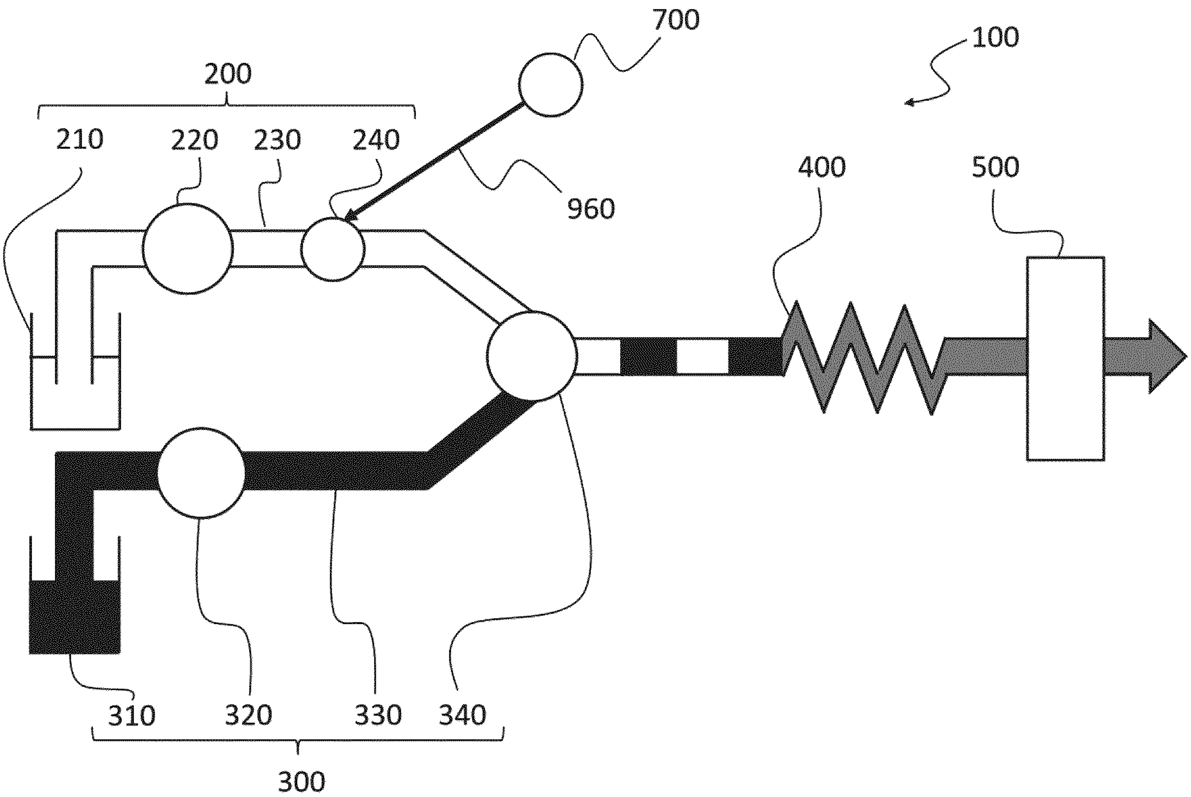
700
100
200
210
220
230
240
960
400
500
310
320
330
340
300

FIG.3

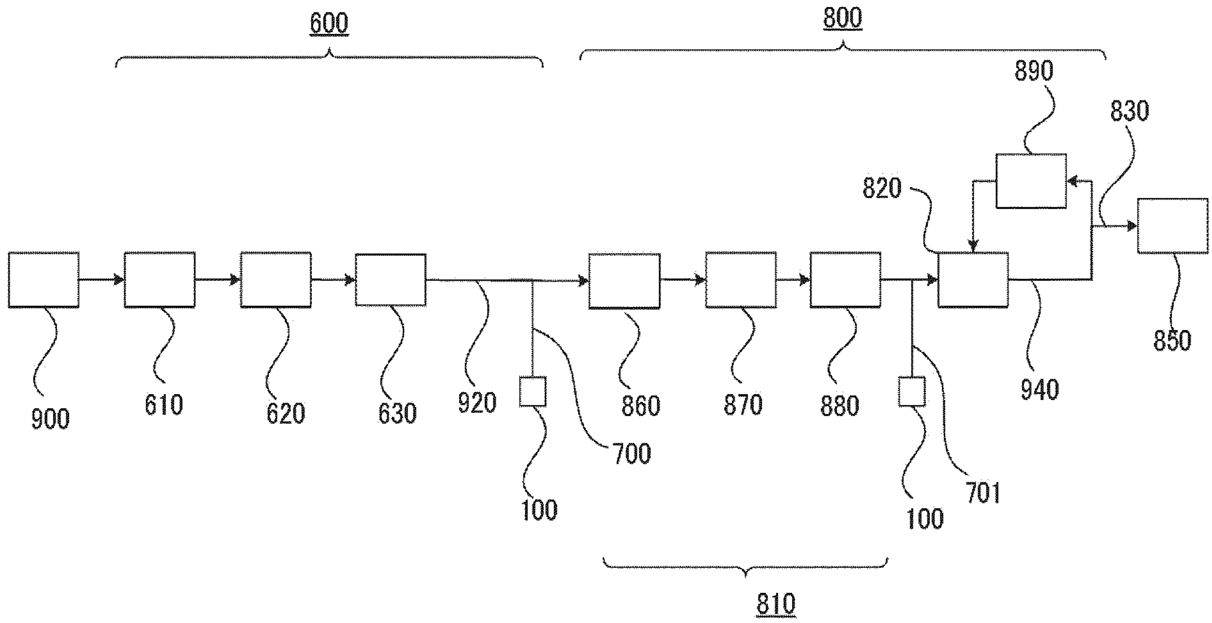
600
800
890
830
820
900
610
620
630
920
700
100
860
870
880
100
701
940
850
810

FIG.4

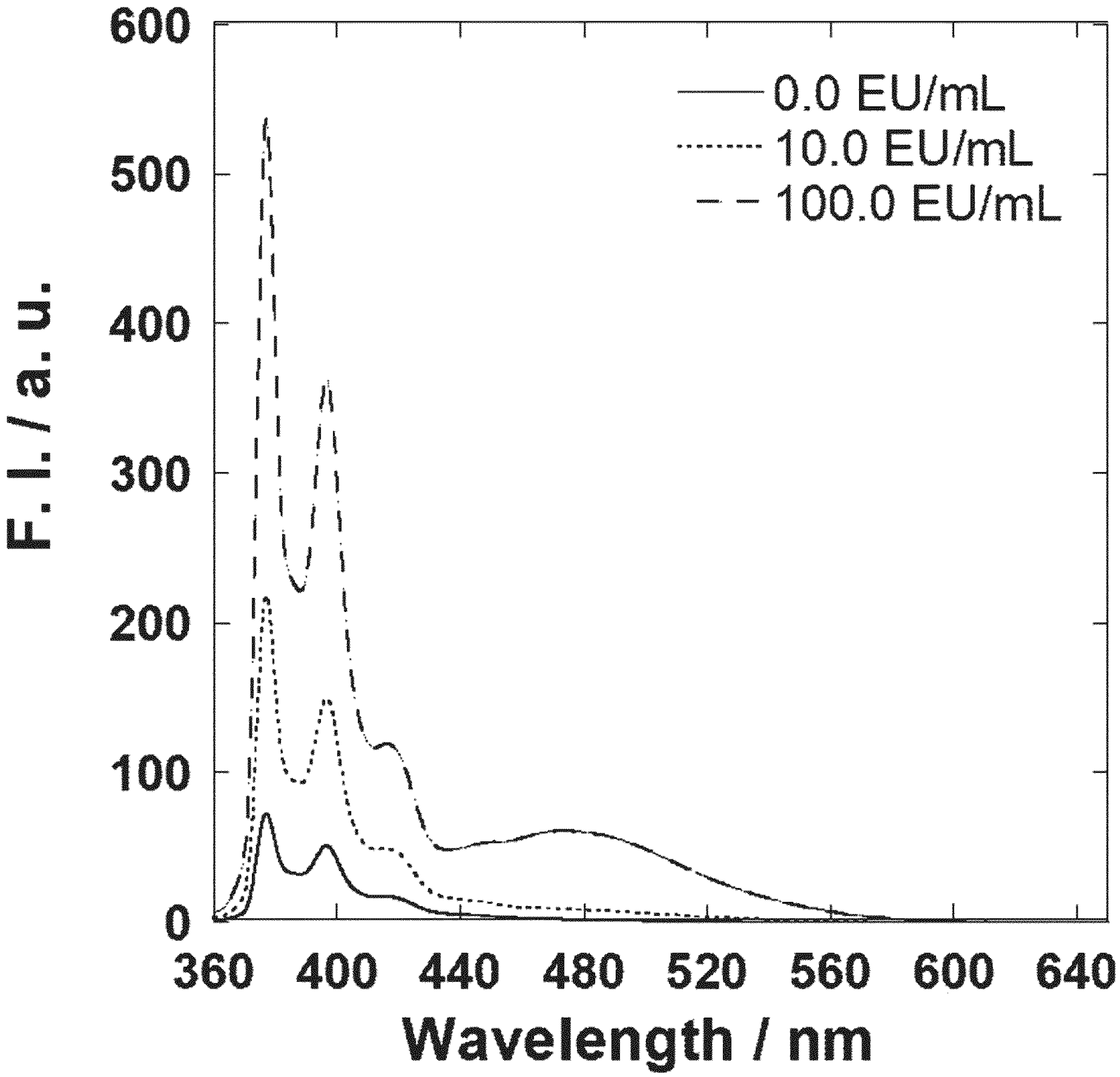
600
500
400
300
200
100
0
F. I. / a. u.
0.0 EU/mL
10.0 EU/mL
100.0 EU/mL
360 400 440 480 520 560 600 640
Wavelength / nm

FIG.5

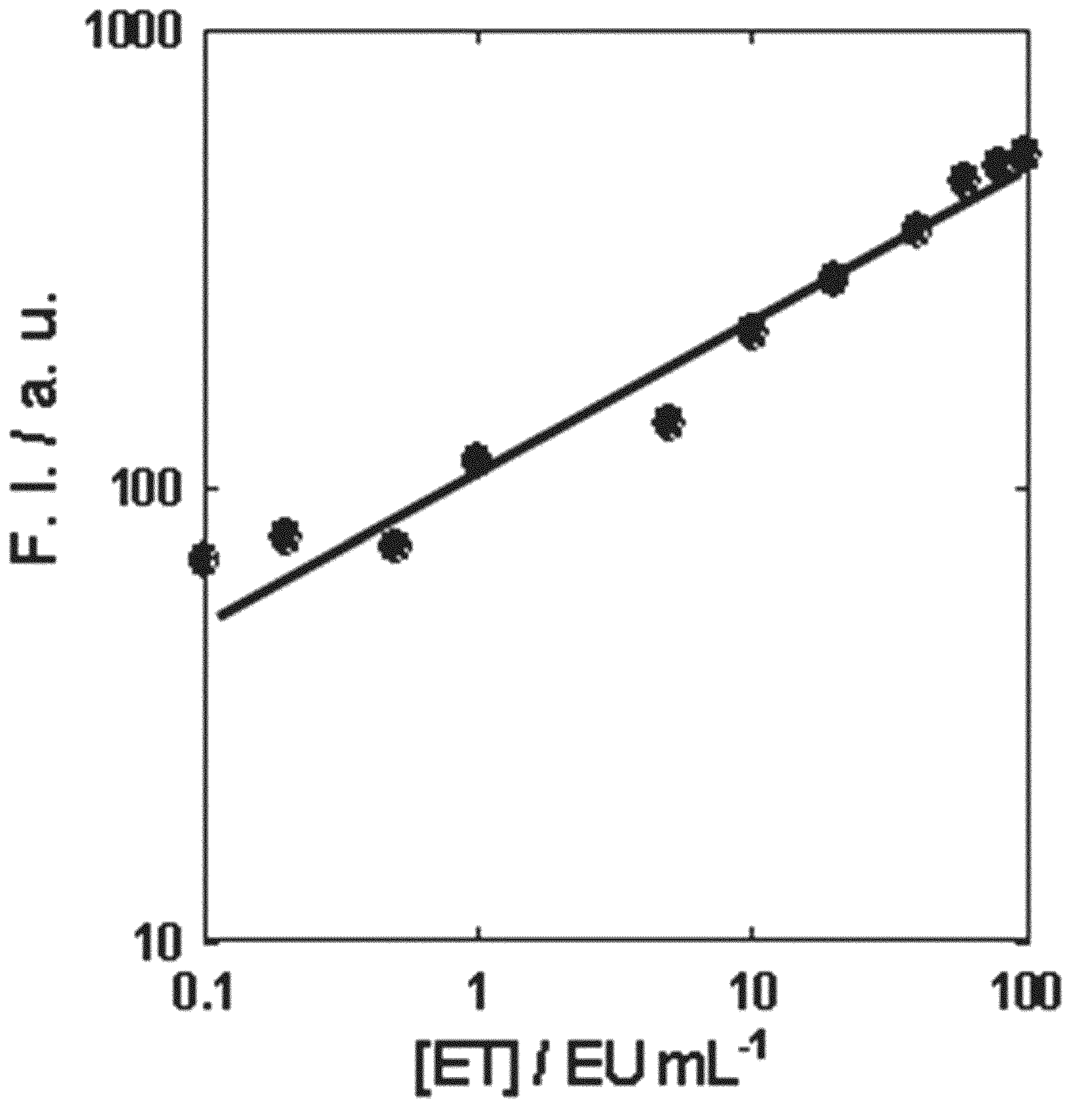

FIG.6

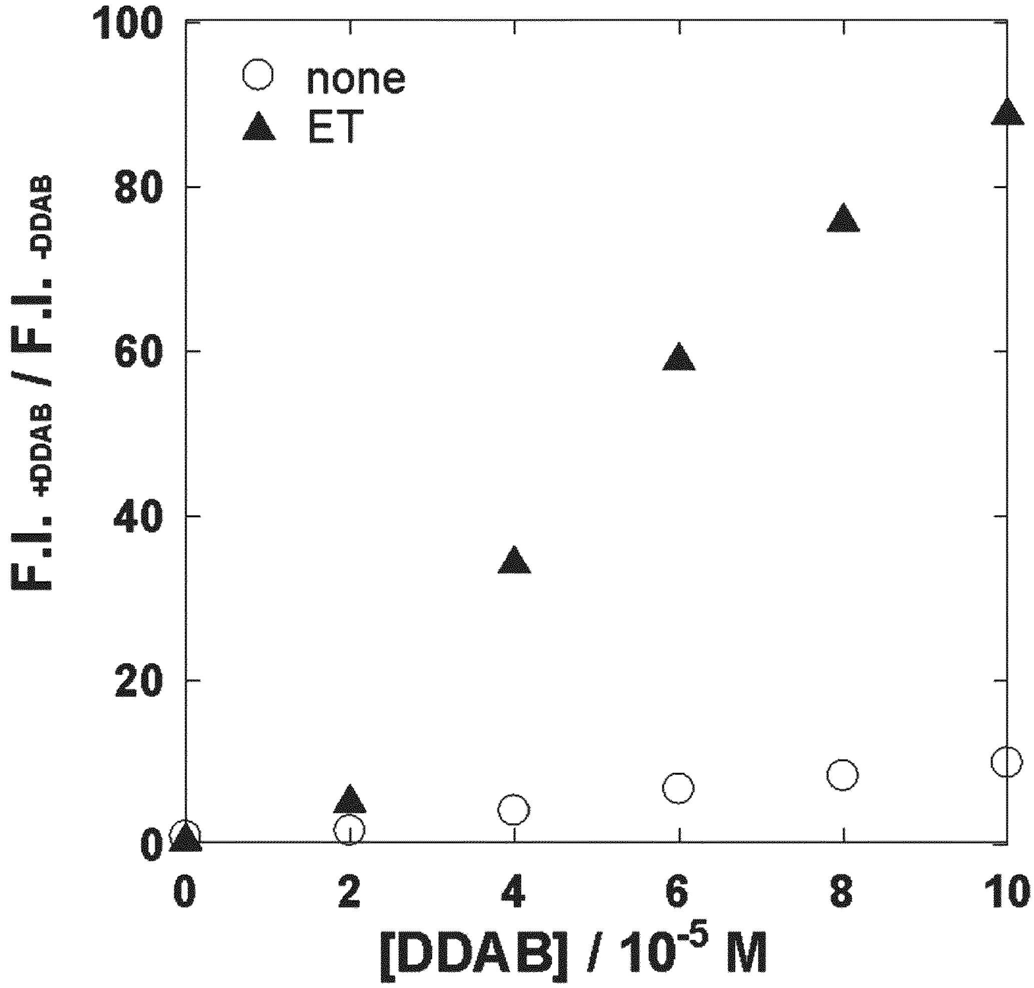

FIG.7

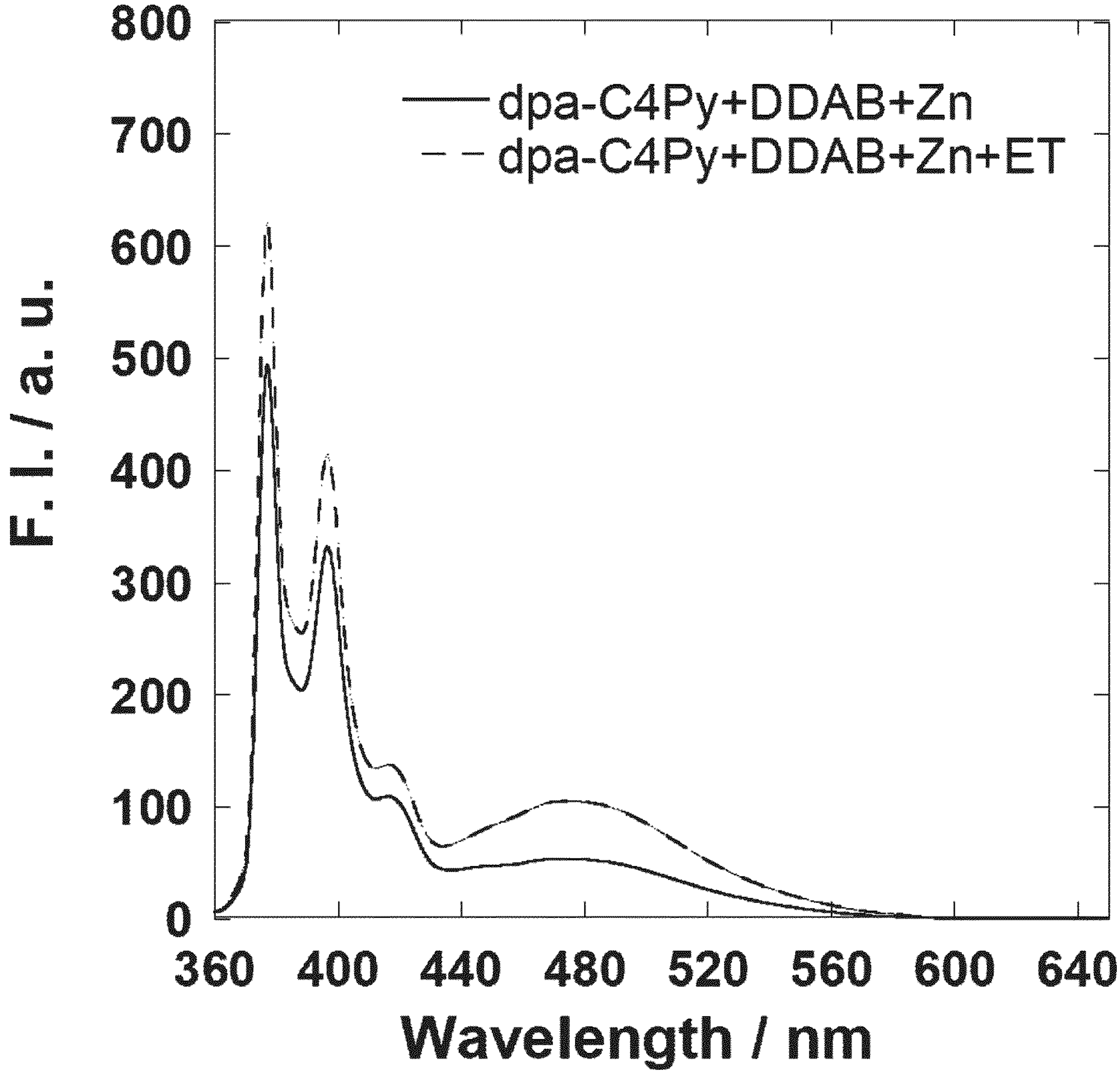
800
700
600
500
400
300
200
100
0
F. I. / a. u.
dpa-C4Py+DDAB+Zn
dpa-C4Py+DDAB+Zn+ET
360
400
440
480
520
560
600
640
Wavelength / nm

FIG.8

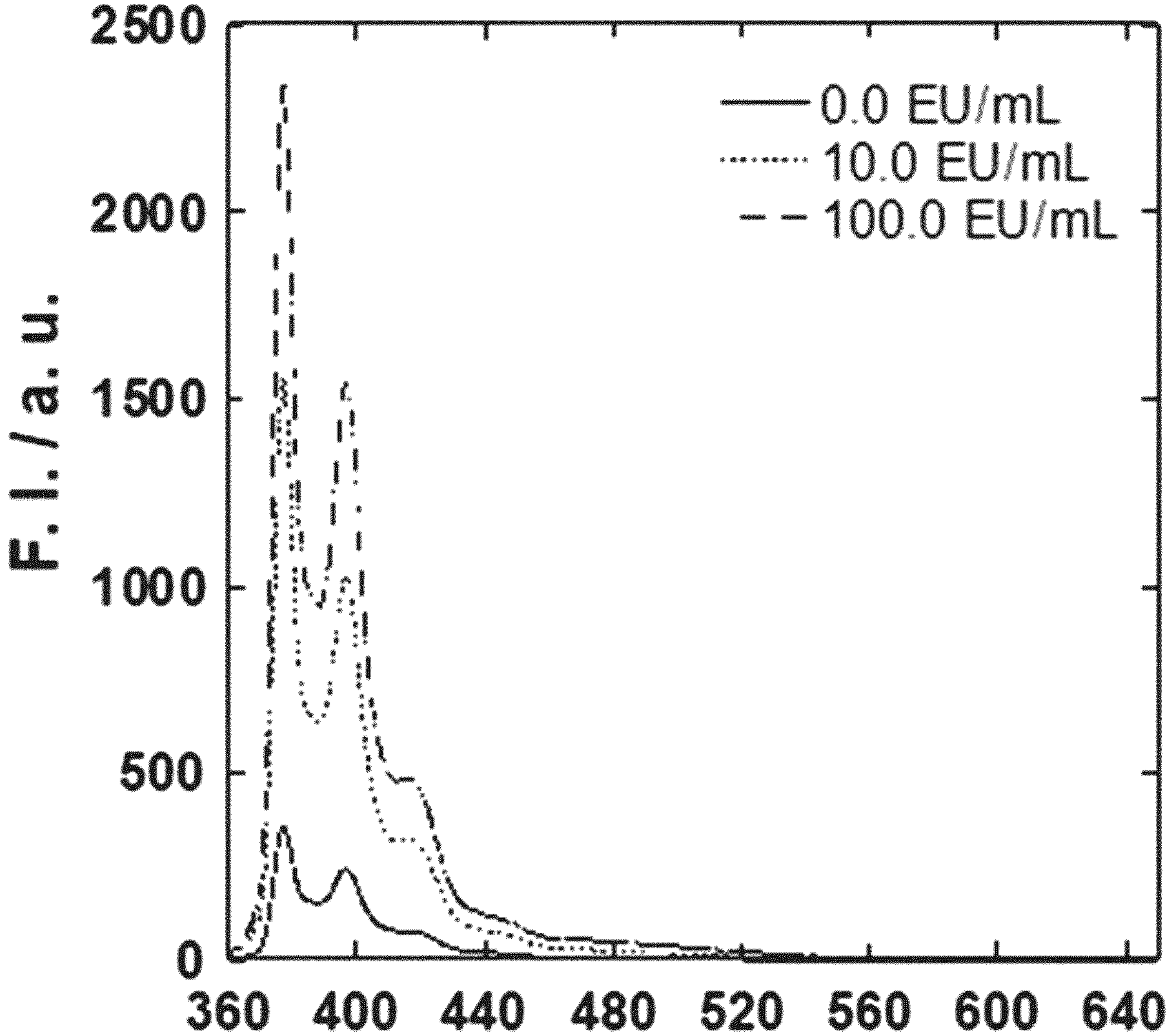
2500
2000
1500
1000
500
0
F. I. / a. u.
360
400
440
480
520
560
600
640
Wavelength / nm
0.0 EU/mL
10.0 EU/mL
100.0 EU/mL

FIG.9

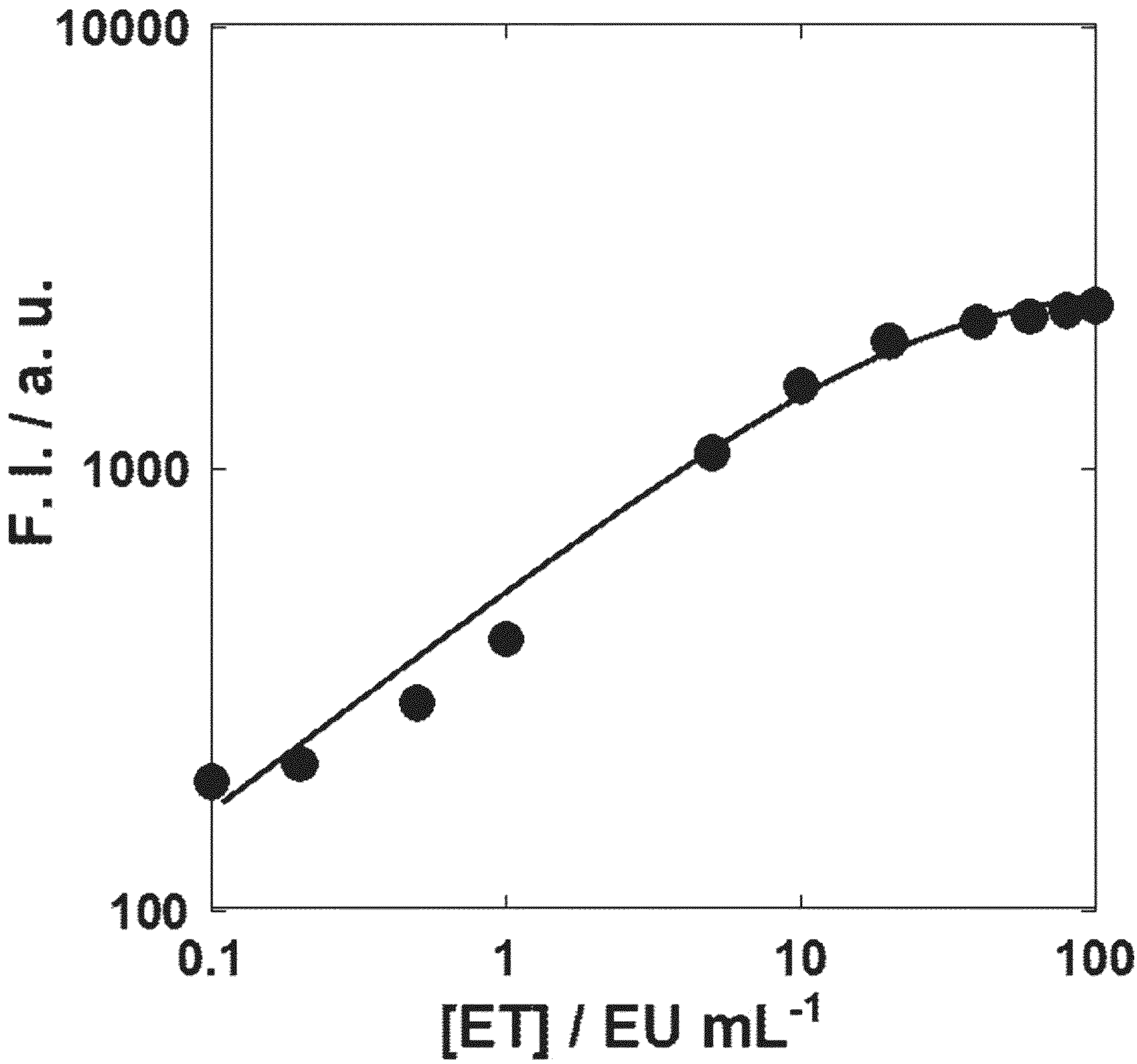

FIG.10

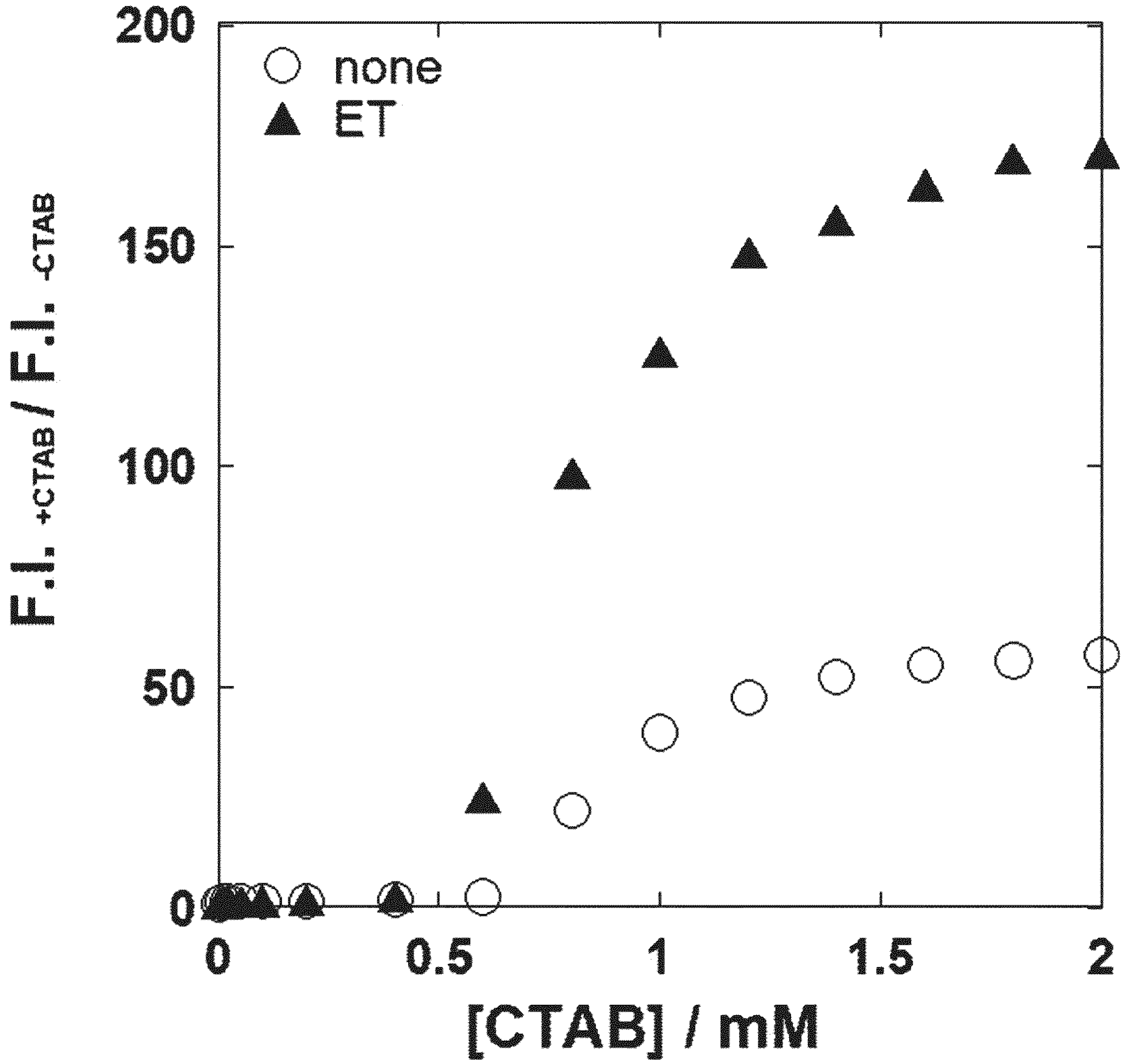

## INTERNATIONAL SEARCH REPORT

International application No.
**PCT/JP2022/009508**

**A. CLASSIFICATION OF SUBJECT MATTER**

***B01D 61/02***(2006.01)i; ***B01D 61/58***(2006.01)i; ***B01D 71/06***(2006.01)i; ***C02F 1/04***(2006.01)i; ***C02F 1/42***(2006.01)i; ***C02F 1/44***(2006.01)i; ***G01N 33/53***(2006.01)i; ***G01N 33/92***(2006.01)i
FI: G01N33/53 S; B01D61/58; B01D71/06; C02F1/44 A; C02F1/44 D; C02F1/44 H; C02F1/44 J; C02F1/42 A; C02F1/42 B; B01D61/02; C02F1/04 Z; G01N33/92 Z

According to International Patent Classification (IPC) or to both national classification and IPC

**B. FIELDS SEARCHED**

Minimum documentation searched (classification system followed by classification symbols)

B01D61/02; B01D61/58; B01D71/06; C02F1/04; C02F1/42; C02F1/44; G01N33/53; G01N33/92

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X<br>Y | TSUCHIDO, Y. et al., Micelle-Type Sensor for Saccharide Recognition by Using Boronic Acid Fluorescence Amphiphilic Probe and Surfactants, Solvent Extraction and Ion Exchange, 12 February 2021, vol. 39, no. 5, 6, pp. 668-677, doi.org/10.1080/07366299.2021.1876988 in particular, abstract, fig. 1 | 1-3, 5-10, 13, 15-20<br>1-20 |
| Y | JP 2007-93378 A (JAPAN SCIENCE & TECHNOLOGY AGENCY) 12 April 2007 (2007-04-12) paragraphs [0002], [0024], [0033] | 11 |
| Y | JI, Y. et al., Development of boronic acid-functionalized mesoporous silica-coated core/shell magnetic microspheres with large pores for endotoxin removal, Journal of Chromatography A, 2019, vol. 1602, pp. 91-99 in particular, fig. 3(a) | 11 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **18 March 2022** | **05 April 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2022/009508**

**C. DOCUMENTS CONSIDERED TO BE RELEVANT**

| Category* | Citation of document, with indication, where appropriate, of the relevant passages | Relevant to claim No. |
|---|---|---|
| X<br><br><br><br><br>Y | ZHANG, L. et al, Fluorescent Binary Ensemble Based on Pyrene Derivative and Sodium Dodecyl Sulfate Assemblies as a Chemical Tongue for Discriminating Metal Ions and Brand Water, ACS Sensors, 2017, vol. 2, pp. 1821-1830<br>in particular, abstract, p. 1822, left column, p. 1826, right column to p. 1828, right column, schemes 1, 2, fig. 3, table 1 | 1-2, 8-10, 21, 24-25<br><br><br><br><br>1-20, 22-23, 26-27 |
| Y | JP 2020-138154 A (NOMURA MICRO SCIENCE CO., LTD.) 03 September 2020 (2020-09-03)<br>paragraph [0002] | 22-23, 26-27 |
| Y | JP 2000-263043 A (DAIICHI PHARMACEUTICAL CO., LTD.) 26 September 2000 (2000-09-26)<br>paragraph [0002] | 22-23, 26-27 |
| X<br><br><br><br>Y | DEY, N. et al., A Glimpse of Our Journey into the Design of Optical Probes in Self-assembled Surfactant Aggregates, The Chemical Record, 2016, vol. 16, pp. 1934-1949, DOI: 10.1002/tcr.201600012<br>in particular, fig. 4, 5 | 1-4, 8-10, 13-14, 18-20<br><br><br><br>1-20 |
| X<br><br><br>Y | US 2016/0304473 A1 (THE GOVERNING COUNCIL OF THE UNIVERSITY OF TORONTO) 20 October 2016 (2016-10-20)<br>claims, examples | 1-11, 13-20<br><br><br>12 |
| Y | JP 2018-535671 A (ARSANIS BIOSCIENCES GMBH) 06 December 2018 (2018-12-06)<br>paragraph [0271] | 11-12 |
| Y | JP 2007-256214 A (AKITA UNIVERSITY) 04 October 2007 (2007-10-04)<br>paragraph [0040] | 11-12 |
| Y | US 2020/0270218 A1 (THE GOVERNING COUNCIL OF THE UNIVERSITY OF TORONTO) 27 August 2020 (2020-08-27)<br>claims, paragraphs [0009], [0141], examples | 1-20 |

Form PCT/ISA/210 (second sheet) (January 2015)

**INTERNATIONAL SEARCH REPORT**
**Information on patent family members**

International application No.
**PCT/JP2022/009508**

| Patent document cited in search report | Publication date (day/month/year) | Patent family member(s) | Publication date (day/month/year) |
|---|---|---|---|
| JP 2007-93378 A | 12 April 2007 | (Family: none) | |
| JP 2020-138154 A | 03 September 2020 | (Family: none) | |
| JP 2000-263043 A | 26 September 2000 | (Family: none) | |
| US 2016/0304473 A1 | 20 October 2016 | WO 2015/089639 A1 | |
| JP 2018-535671 A | 06 December 2018 | US 2018/0312576 A1<br>paragraph [0409]<br>WO 2017/071835 A1<br>KR 10-2018-0083869 A<br>CN 108699137 A | |
| JP 2007-256214 A | 04 October 2007 | (Family: none) | |
| US 2020/0270218 A1 | 27 August 2020 | WO 2019/068177 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2019025456 A
- JP 2016151482 A
- JP 2007093378 A


### Non-patent literature cited in the description


- **KENICHI TANAMOTO.** Endotoxin and the Quality Control of Medicine. *Bull. Natl. Inst. Health Sci.,* 2008, vol. 126, 19-33